# EUROPÄISCHE PATENTSCHRIFT

(11) **EP 3 139 869 B1**
(45) Veröffentlichungstag und Bekanntmachung des Hinweises auf die Patenterteilung: **20.11.2019**
(21) Anmeldenummer: 15721125.1
(22) Anmeldetag: 06.05.2015
(51) Int. Cl.: A61F 2/66, A61F 2/50

(54) **PROTHESENFUSS**
PROSTHETIC FOOT
PROTHÈSE DE PIED

(30) Priorität: 07.05.2014 DE 102014006571
(43) Veröffentlichungstag der Anmeldung: 15.03.2017
(73) Patentinhaber: Ottobock SE & Co. KGaA, 37115 Duderstadt (DE)
(72) Erfinder: PUSCH, Martin, 37115 Duderstadt (DE); NÖRTHEMANN, Jens, 37115 Duderstadt (DE); RANE, Darshan, Milcreek 84106 (US); MÖNICKE, Carsten, 37115 Duderstadt (DE); WILLIAMS, Nathan Aaron, Meridan 82801 (US)
(74) Vertreter: Gramm, Lins & Partner Patent- und Rechtsanwälte PartGmbB
(86) Internationale Anmeldenummer: PCT/EP2015/000926
(87) Internationale Veröffentlichungsnummer: WO 2015/169443

(56) Entgegenhaltungen:
- EP-A1- 2 420 212
- WO-A2-2012/009319
- US-A1- 2004 068 327
- US-A1- 2005 033 450
- US-A1- 2013 144 403

## Beschreibung

Die Erfindung betrifft einen Prothesenfuß mit einem Strukturbauteil mit proximalen Anschlussmitteln zur Befestigung des Prothesenfußes an einem Unterschenkelrohr, Unterschenkelschaft oder einem Prothesenkniegelenk, einem an dem Strukturbauteil festgelegten oder ausgebildeten Vorderfußabschnitt und einem dem Strukturbauteil zugeordneten, fersenseitigen Feder-Dämpfersystem, das bei einem Fersenauftritt komprimiert wird und sich auf einen sohlenseitigen Führungselement abstützt. Der Prothesenfuß ist auch zur Anordnung in einem Schuh geeignet und dazu ausgebildet.

Prothesenfüße dienen als distaler Abschluss einer Protheseneinrichtung und können an einem Unterschenkelrohr, das an einem Prothesenkniegelenk befestigt ist, an einem Prothesenschaft direkt oder an dem Prothesenkniegelenk festgelegt werden. Dazu sind an dem Prothesenfuß regelmäßig Anschlussmittel am proximalen Ende vorgesehen, um einen stabilen und dauerhaften Anschluss an der proximalen Prothesenkomponente herzustellen. Prothesenfüße werden üblicherweise mit einer Kosmetik versehen, die aus einem Kunststoff besteht und der Form eines natürlichen Fußes angenähert ausgebildet ist.

Die konstruktiv einfachste Form eines Prothesenfußes ist ein starrer Fuß, der jedoch signifikante Nachteile hinsichtlich der elastischen Eigenschaften oder der Abrolleigenschaften hat.

Zur Dämpfung des Impulses beim Fersenauftritt können Dämpferelemente oder Fersenfedern vorgesehen sein, ebenfalls ist es möglich, dass im Vorderfußbereich eine Feder angeordnet ist, um das Abrollen des Fußes über die gesamte Standphase hinweg zu erleichtern und darüber hinaus in der terminalen Standphase die vorher aufgenommene Verformungsenergie wieder abzugeben, um den Prothesenfußnutzer beim Gehen zu unterstützen.

Die US 7,172,630 B2 betrifft einen Prothesenfuß mit zwei Blattfederelementen, die im Vorderfußbereich miteinander gekoppelt sind. An einer Blattfeder ist ein Exzenter angeordnet, der gegen eine Spannfeder drückt. Durch Verlagerung des Exzenters kann die Spannfeder gespannt oder entspannt werden, so dass im Laufe der Abrollbewegung ein bestimmtes Kraftprofil realisiert werden kann.

Die US 5,139,525 A betrifft einen Prothesenfuß mit einer gelenkigen Aufnahme für ein Unterschenkelrohr. Die gelenkige Aufnahme ist im Bereich des natürlichen Knöchelgelenks angeordnet. Die Federcharakteristik des Prothesenfußes kann sich über den Verlauf einer Abrollbewegung verändern.

Die US 2007/0061016 A1 betrifft einen Prothesenfuß mit einer Fersenplatte und einer Zehenplatte, die um eine Zentralachse schwenkbar gelenkig miteinander verbunden sind. Eine Feder ist zwischen der Fersenplatte und der Zehenplatte angeordnet und stützt sich an einer rückwärtigen Verlängerung der Zehenplatte ab. Eine Adapterplatte ist ebenfalls schwenkbar um die zentrale Achse angeordnet. Über Sensoreinrichtungen wird aktiv die beim Gehen auftretende Energie gespeichert und wieder freigegeben.

Die US 6, 719,807 B2 betrifft einen Prothesenfuß mit wellenartig geschwungenen Vorfußfeder und einer im Mittelfußbereich befestigten, sich nach hinten erstreckenden Fersenfeder, die in einer Fußkosmetik angeordnet ist. Vorfußfeder und Fersenfeder sind in einem Rahmen gelagert. Eine Variante sieht vor, dass die Fersenfeder mit der Vorfußfeder über eine Basisfeder verbunden ist, die einerseits an dem posterioren Ende der Fersenfeder und andererseits im Mittelfußbereich an der Vorfußfeder befestigt ist.

Die US 2012/0046760 A1 betrifft einen Prothesenfuß mit einer einteiligen Feder, die einen unteren Basisabschnitt und ein bogenförmig nach oben ansteigendes Oberteil aufweist. Basisabschnitt und Oberteil sind im Vorderfußbereich mit einem Längsschlitz versehen, im Fersenbereich ist ein Dämpfer aus einem Elastomermaterial angeordnet.

US 2014/0046456 A1 betrifft einen Prothesenfuß mit einer ebenen Basisfeder, einer bogenförmigen Vorfußfeder mit daran befestigtem Anschlussadapter und einem Dämpferelement, das im Bereich der Ferse die Basisfeder gegen die Vorfußfeder abstützt.

Die Chas A Blatchford & Sons Ltd. vertreibt unter der Bezeichnung "endolite Blade XT" einen Prothesenfuß für Breitensportler und Läufer mit einer Vorderfußfeder, die einen im Wesentlichen waagerechten Kopfabschnitt und eine nach außen konvex geformte, einteilige Feder aufweist, die im unteren Bereich durch einen Schlitz zweigeteilt ist. In dem vorderen Bereich der Feder ist ein Sohlenschutz und eine Fersenfeder über zwei Schrauben befestigt, über einen Keil kann die Federhärte der Fersenfeder eingestellt werden. Das Einsetzen des Fersenkeils hat zur Folge, dass die Fersenfeder weniger flexibel reagiert.

US 2005/0033450 A1 stellt den nächstliegenden Stand der Technik dar und betrifft einen Prothesenfuß mit einem langgestreckten Vorderfußteil der sich nach hinten durch einen oberen Befestigungsabschnitt erstreckt, wobei der Befestigungsabschnitt ausgebildet ist, um mit einer Gliedmaße eines Patienten gekoppelt zu werden. Nach unten erstreckt sich der Vorderfußteil durch einen Knöchelbereich, der im Bereich eines natürlichen Knöchels des natürlichen Fußes angeordnet ist. Nach vorne erstreckt sich der Vorfußabschnitt durch einen Bogenbereich bis zu einem Zehenbereich im Bereich der Zehen eines natürlichen Fußes. Weiterhin weist der Prothesenfuß einen langgestreckten hinteren Knöchelbereich auf, der sich nach hinten durch einen oberen Befestigungsabschnitt erstreckt, der an dem Befestigungsabschnitt des Vorderfußteils befestigt ist. Weiterhin erstreckt sich der rückwärtige Knöchelbereich nach unten durch einen Knöchelabschnitt, nach vorne unter den Knöchelabschnitt des Vorderfußteils und nach hinten in Richtung auf einen Fersenabschnitt im Bereich der Ferse eines natürlichen Fußes. Eine untere Fußplatte ist unterhalb des Vorderfußbereiches und des Knöchelbereiches angeordnet und erstreckt sich im Wesentlichen von dem Zehenabschnitt zu dem Fersenabschnitt. Eine Höhe umgibt zumindest Teile des oberen Vorfußteils, des Knöchelbereichs und der unteren Fußplatte.

Aufgabe der vorliegenden Erfindung ist es, einen Prothesenfuß bereitzustellen, der einfach aufgebaut ist, das Gehen für einen Prothesenfußnutzer erleichtert und insbesondere bei sportlichen Aktivitäten vorteilhaft einsetzbar ist.

Erfindungsgemäß wird diese Aufgabe durch einen Prothesenfuß mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Der erfindungsgemäße Prothesenfuß mit einem Strukturbauteil mit proximalen Anschlussmitteln zur Befestigung des Prothesenfußes an einem Unterschenkelrohr, einem Unterschenkelschaft oder einem Prothesenkniegelenk, einem an dem Strukturbauteil festgelegten oder ausgebildeten Vorderfußabschnitt und einem dem Strukturbauteil zugeordneten, fersenseitigen Feder-Dämpfersystem, das bei einem Fersenauftritt komprimiert wird und sich auf einem sohlenseitigen Führungselement abstützt sieht vor, dass das Strukturbauteil als eine Blattfeder ausgebildet ist, die sich von den proximalen Anschlussmitteln in posteriore Richtung erstreckt, einen Bogen ausbildet und in anteriore und distale Richtung geführt ist, wobei der Bogen posterior über das Führungselement übersteht. Durch das fersenseitige Feder-Dämpfer-System, das über das Führungselement mit der Stützstruktur verbunden ist, ist es möglich, bei einer Fersenbelastung eine Führung der Verlagerungsbewegung des Feder-Dämpfer-Systems und des dem Feder-Dämpfer-Systems zugeordneten Führungselementes zu ermöglichen. Dadurch wird eine Pronations- und Supinationsbewegung beim Aufsetzen zumindest vermindert, wodurch eine präzisere Führung des Prothesenfußes während des Gehens realisiert werden kann. Darüber hinaus findet beim Abrollen von der Ferse auf den Vorderfuß eine Übertragung der in dem Feder-Dämpfer-System gespeicherten Energie auf das Strukturbauteil dadurch statt, dass das Feder-Dämpfer-System entspannt wird, Energie freigibt und dadurch eine Verzögerung einer Verformung des Strukturbauteiles bewirkt. Durch die Ausgestaltung des Strukturbauteils als Blattfeder ist es möglich, die beim Gehen, insbesondere beim Laufen aufgenommene Energie zu speichern und einen langen Verformungsweg bereitzustellen, so dass eine große Energiespeicherfähigkeit während der Standphase bereitgestellt werden kann. Durch den nach hinten über das Führungselement überstehenden Bogen ergibt sich eine signifikante Verlängerung der effektiven Federlänge, so dass ein hohes Maß an Verformung und Verformbarkeit des Strukturbauteils in Gestalt der Blattfeder gegeben ist. Der Bogen erlaubt es darüber hinaus, trotz langer effektiver Federlänge eine geringe Prothesenfußhöhe zu erzielen und gleichzeitig durch das nach hinten Hinausstehen im Bereich des natürlichen Knöchelgelenkes die Tragbarkeit im Schuh gewährleistet.

Der Bogen des Strukturbauteils ragt über die natürliche Knöchelposition in posteriore Richtung hinaus und ist ungefähr in der Höhe der natürlichen Knöchelposition oder etwas darüber hinausgehend angeordnet, so dass der Scheitelpunkt des Bogens hinter dem posterioren, hinteren Ende des Führungselementes liegt. Das Führungselement ist vorteilhafterweise an der Unterseite des Feder-Dämpfersystems angeordnet und als Blattfeder im Vorderfußabschnitt des Strukturbauteils fest daran gelagert. Das Führungselement ist dabei vorteilhafterweise dünner als die Blattfeder des Strukturbauteils mit dem Vorderfußabschnitt, gegebenenfalls als Blattfeder ausgebildet oder einstückig an dem Strukturbauteil angeformt sein kann, ausgebildet, um eine Vorspannung des Vorderfußabschnittes bei einem Fersenauftritt nach Möglichkeit zu verringern. Durch die dünne Ausgestaltung des Führungselementes wird eine größere Verformbarkeit bei einem Fersenstoß gewährleistet, so dass nur ein geringer Kraftanteil und Energieeintrag in den Vorderfußabschnitt eingeleitet wird. Das Führungselement kann um eine quer zur Längserstreckung des Prothesenfuß orientierte Achse freibeweglich oder nahezu freibeweglich an der Stützstruktur gelagert werden, beispielsweise indem eine Art Scharnier in dem Führungselement ausgebildet ist. Dabei wird unter einer Achse nicht ein Schienenelement verstanden, sondern eine gedachte Linie, an die sich Komponenten zueinander verschwenken lassen. Eine Achse liegt beispielsweise auch dann vor, wenn ein Filmscharnier, ein Elastomerelement oder eine polyzentrische Lagerung vorgesehen ist, über die zwei Komponenten miteinander gekoppelt sind. Zur Ausbildung einer Schwenkachse kann das Führungselement ein Filmscharnier aufweisen, das einerseits ein klappenartiges Verschwenken um eine waagerechte Achse ermöglich, andererseits eine Torsion um die Fußlängsachse und die Fußhochachse verhindert oder vermindert.

Die Lagerung des Führungselementes kann unmittelbar an dem Strukturbauteil erfolgen, so dass die Achse, um die das Führungselement oder Teile des Führungselementes frei beweglich oder nahezu frei beweglich ist, unmittelbar an der Stützstruktur festgelegt ist oder durch das Führungselement ausgebildet wird.

Neben der Ausgestaltung des Führungselementes als eine Blattfeder kann dieses auch laschenartig oder klappartig ausgebildet sein, wodurch eine Krafteinleitung in den Vorderfußabschnitt weiter verringert wird.

Das Feder-Dämpfer-System ist vorteilhafterweise als Schaumstoffelement oder Elastomerelement ausgebildet, wodurch es möglich ist, mit preiswerten Materialien eine Vielzahl von Formen bereitzustellen, die eine jeweils unterschiedliche Feder-Dämpfer-Charakteristik aufweisen, so dass ein leichtes Anpassen des Prothesenfußes an die Wünsche oder Notwendigkeiten des Prothesenfußnutzers möglich ist. Auch ist es möglich, unterschiedliche Absatzhöhen leicht zu realisieren. Durch einen Materialmix können gewünschte Feder- und Dämpfungseigenschaften preiswert eingestellt werden. Durch die Ausgestaltung des Feder-Dämpfer-Systems als Schaumstoffelement oder Elastomerelement ist es möglich, nur geringe oder keine Scherkräfte aufzubauen, so dass keine Zugkräfte oder Druckkräfte in das Führungselement eingeleitet werden. Vorteilhafterweise stützt sich das Feder-Dämfer-System im Bereich des natürlichen Knöchels an der Unterseite des Strukturbauteils ab, vorteilhafterweise in einem Bereich, an dem der Bogen beginnt, so dass bei einer Abstützung im Übergang von dem Bogen zu dem Vorderfußabschnitt eine Abstützung erfolgt. Dadurch wird weiter verhindert, dass ein Energieeintrag beim Fersenstoß in den Vorderfußabschnitt erfolgt.

Das Feder-Dämpfer-System kann reversibel zwischen der Unterseite der Stützstruktur und der Oberseite des Führungselementes angeordnet sein, beispielsweise durch ein an der Unterseite des Strukturbauteils befestigten, bevorzugt angeklebten Halter, in den formschlüssig das Feder-Dämpfer-System aus einem Schaumstoffelement oder einen Elastomerelement eingesetzt werden kann. An der Oberseite des Führungselementes kann ebenfalls ein Formschlusselement angeordnet sein, so dass durch einfaches Einschieben und Einrasten des Feder-Dämpfer-Systems eine Anpassung an die gewünschte Steifigkeit oder ein Auswechseln bei Wartungsarbeiten erfolgen kann.

Das Führungselement ist vorteilhafterweise als eine Lasche oder Klappe ausgebildet und kann aus einem Metall, insbesondere Leichtmetall oder einem Kunststoffmaterial ausgebildet sein. Die Schwenkachse, an der das Führungselement in Gestalt einer Lasche oder Klappe gelagert ist, liegt dabei an der Stützstruktur, so dass eine relativ große Länge für das Führungselement realisiert werden kann, wodurch das Feder-Dämpfer-System präzise eingestellt werden kann.

An der Unterseite des Führungselementes kann ein Sohlenelement aus einem elastischen Material angeordnet sein, das durch seine geometrischen Abmessungen und Festigkeitseigenschaften eine zusätzliche Feder und Dämpfung bereitstellt, so dass das Feder-Dämpfer-System zwischen der Stützstruktur und dem Führungselement nicht das einzige nachgiebige Element ist. Durch die Eigenelastizität des Sohlenelementes kann eine schlagartige Lastaufnahme und Lastübertragung auf das Feder-Dämpfer-System vermieden und gedämpft werden.

An der Unterseite des Führungselementes kann ein Sohlenelement aus einem elastischen Material angeordnet sein, das durch seine geometrischen Abmessungen und Festigkeitseigenschaften so ausgebildet ist, dass das COP (center of pressure) oder der Kraftangriffspunkt möglichst lange beim Abrollen im Fersenbereich verbleibt, um ein vorzeitiges Aufladen oder eine Verformung der Vorfußfeder zu vermeiden.

Der Vorderfußabschnitt kann starr an dem Strukturbauteil festgelegt sein und ist vorteilhafterweise als Blattfeder ausgebildet oder weist zumindest eine Blattfeder oder ein Blattfederelement auf. Durch die starre Anbindung an die Stützstruktur ist es möglich, eine präzise Führung des Vorderfußabschnittes zu erreichen, wodurch die Kontrolle über den Prothesenfuß beim Gehen erleichtert wird. Die Ausgestaltung des Vorderfußabschnittes als Feder ermöglicht es, während des Gehens Energie aufzunehmen. Darüber hinaus ist es möglich, nach dem Auftreten mit dem Vorderfußabschnittes nach dem Fersenstoß von dem Feder-Dämpfer-Element die gespeicherte Energie auf den Vorderfuß zu übertragen, wobei die Gesamthöhe des Prothesenfußes konstant bleibt, so dass die Kraftleitung über den Boden bzw. die Sohlenstruktur erfolgt. Die in dem Feder-Dämpfer-Element gespeicherte Energie wird dann über den Vorderfußabschnitt abgegeben und erleichtert das Abdrücken des Nutzers am Ende der Standphase.

Zwischen dem Führungselement und der Stützstruktur kann ein Überlastanschlag angeordnet sein, um zu verhindern, dass bei einem massiven Energieeintrag das Feder-Dämpfer-System zu weit komprimiert wird, wodurch eine Instabilität während des Gehens entstehen kann.

Das Führungselement kann um eine in anterior-posterior-Richtung verlaufende Achse und/oder eine in proximal-distal-Richtung verlaufende Achse unverlagerbar gelagert sein, so dass eine Ausweichbewegung des Führungselementes nicht möglich ist und insbesondere eine Supination und Pronation des Führungselementes nach dem Aufsetzen verhindert wird. Die starre Lagerung um eine Achse, die in anterior-posterior Richtung verläuft, verhindert, dass die Ferse des Prothesenfußes beim Gehen seitlich wegknickt, was insbesondere bei einem Einsatz als Sportfuß vorteilhaft ist.

An dem freien Ende des Führungselementes kann ein Absatz in proximaler Richtung oder eine konvexe Wölbung ausgebildet sein, um ein leichtes Abrollen sowie eine Anpassung der Sohlenstruktur, beispielsweise an das Körpergewicht oder an die Gangart des Patienten, zu ermöglichen.

Das Strukturbauteil selbst kann elastisch ausgebildet sein, insbesondere einteilig oder einstückig ausgebildet sein, so dass sich eine durchgehende Blattfeder von den proximalen Anschlussmitteln bis zum vorderen Ende des Strukturbauteils, insbesondere bis zum vorderen Ende des Prothesenfußes ergibt.

An dem posterioren Ende des Führungselementes und der Unterseite des Strukturbauteils kann je ein Formschlusselement zur Festlegung des Feder-Dämpfer-Systems festgelegt sein, insbesondere sind die Formschlusselemente angeklebt. Das an dem Führungselement angeordnete Formschlusselement kann als Fersenkappe und als posteriorer unterer Abschluss des Prothesenfußes ausgebildet sein und gleichzeitig eine Dämpfung oder Polsterung im Fersenbereich sowie eine Profilierung bereitstellen, sofern der Prothesenfuß nicht in einem Schuh getragen werden sollte, so dass vor dem Anziehen des Schuhs eine Rutschgefahr vermindert wird.

Auch das obere Formschlusselement zur formschlüssigen Verriegelung an der Unterseite des Strukturbauteils kann daran festgelegt oder festgeschraubt sein, so dass eine einfache Montage und die Montage des Feder-Dämpfer-Systems, das einstückig ausgebildet sein kann und aus unterschiedlichen Materialien bestehen kann, erfolgen kann.

Der nach hinten über das Führungselement überstehende Bogen des Strukturbauteils steht in anterior-posteriorer Richtung über das Feder-Dämpfer-System um eine Strecke hinaus, die zwischen 10% und 30%, insbesondere bevorzugt zwischen 12,5% und 25% der Fußlänge liegt. Die Fußlänge ist dabei diejenige Länge des Prothesenfußes, die von dem vorderen, anterioren Ende bis zum hinteren, posterioren Ende des Führungselemente, gegebenenfalls bis zum hinteren Ende des an dem Führungselement angeordneten Formschlusselementes gemessen wird.

An der Oberseite des Strukturbauteils kann im Vorderfußbereich eine konturbildende Polsterung angeordnet sein, durch die es möglich ist, einen Schuh, in den der Prothesenfuß eingesetzt wird, formbildend auszufüllen. Es ist somit nicht notwendig, dass eine weitere Kosmetik um den Prothesenfuß herum angelegt wird. Vielmehr kann der erfindungsgemäße Prothesenfuß unmittelbar in einem Schuh getragen werden.

In einer Weiterbildung der Erfindung ist vorgesehen, dass an dem Prothesenfuß eine Sohle befestigt ist, so dass dieser auch ohne Schuh getragen werden kann.

Von den proximalen Anschlussmitteln verläuft das Strukturbauteil in dem Bogen konkav gekrümmt, in dem Bereich der Abstützung des Feder-Dämpfer-Systems an der Unterseite des Strukturbauteils liegt eine konvexe Krümmung vor, die vorteilhafterweise im Vergleich zu dem Bogen einen größeren Krümmungsradius aufweist. An den konvexen Krümmungsbereich schließt wiederum eine konkave Krümmung in dem Vorderfußabschnitt an, wobei die Sichtweise auf das Strukturbauteil ausgehend von einer Draufsicht im Bereich der proximalen Anschlussmittel auf der ursprünglichen Oberfläche über den Verlauf von den oberen Anschlussmitteln bis zur Fußspitze verbleibt, also zunächst von oben nach unten schauend und nach Ende des Bogens von unten nach oben schauend.

Das Führungselement kann als eine Blattfeder mit einer von der Ferse bis zur Fußspitze konkav, konvex, konkav verlaufenden Form ausgebildet sein, um einerseits das natürliche Fußgewölbe nachzubilden und andererseits ein sanftes Abrollen nach dem Fersenauftritt, eine hohe Elastizität durch die konvexe Bewegung im Mittelfußbereich und ein sanftes Auftreten und Abrollen im Vorderfußbereich zu gewährleisten. Die Sichtweise ist hierbei von der Unterseite des Führungselementes.

Eine Weiterbildung der Erfindung sieht vor, dass der Vorderfußabschnitt und das Führungselement über eine Ausrichteeinrichtung einander zugeordnet sind. Die Ausrichteeinrichtung stellt gleichzeitig eine Form für eine Klebeverbindung und darüber hinaus einen Schutz der Komponenten vor äußeren Einflüssen oder des Schuhs vor den gegebenenfalls scharfkantigen Blattfedern zur Verfügung, da die Ausrichteinrichtung zusammen mit dem Strukturbauteil und dem Führungselement verklebt wird und an dem Prothesenfuß verbleibt.

Der Vorderfußabschnitt, das Führungselement und die Ausrichteeinrichtung können miteinander verklebt sein und dadurch dauerhaft miteinander befestigt werden.

Der Prothesenfuß ist zur Anordnung in einem Schuh ausgebildet und geeignet, insbesondere durch die Ausgestaltung des Strukturbauteils mit dem nach hinten verlängerten Bogen im Bereich oder oberhalb der natürlichen Knöchelposition und dem Führungselement, das einen Siöhlenfunktion während der Standphase einnimmt.

Nachfolgend wird ein Ausführungsbeispiel der Erfindung anhand der Figuren näher erläutert. Es zeigen:
- Figur 1 -: eine perspektivische Ansicht eines Prothesenfußes;
- Figur 2 -: eine Seitenansicht der Figur 1;
- Figur 3 -: eine Variante mit abgewandeltem Strukturbauteil;
- Figur 4 -: eine Variante der Figur 3;
- Figur 5 -: eine schematische Darstellung einer Seitenkraftkompensation;
- Figur 6: eine Seitenansicht einer orthopädietechnischen Komponente während der Fertigung;
- Figur 7: eine perspektivische Ansicht mit Zuführ- und Auslasseinrichtungen;
- Figur 8: eine Schnittansicht eines Teils einer orthopädietechnischen Komponente;
- Figur 9: eine perspektivische Ansicht einer Ausrichteeinrichtung;
- Figur 10: eine andere Ansicht der Ausrichteeinrichtung aus Figur 9;
- Figur 11: eine Teildarstellung einer orthopädietechnischen Komponente von schräg hinten;
- Figur 12: eine Variante der Figur 11;
- Figur 13: eine perspektivische Teildarstellung einer zweiten Ausführungsform;
- Figur 14: eine andere Ansicht der Ausführungsform aus Figur 13;
- Figur 15: eine Gesamtansicht einer Ausrichteeinrichtung der zweiten Ausführungsform;
- Figur 16: eine Schnittdarstellung der Figur 15;
- Figur 17: eine Schnittdarstellung der Figur 14; sowie
- Figur 18: eine andere Ansicht der Figur 17.

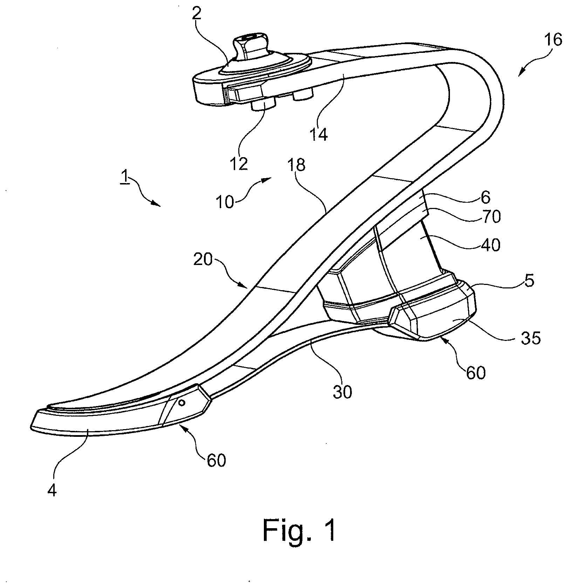

Figur 1 zeigt in einer perspektivischen Seitenansicht einen Prothesenfuß 1 mit einem Strukturbauteil 10 in Gestalt einer einteiligen Blattfeder, an deren proximalen Ende Anschlussmittel 2 in Gestalt eines Adapterpylons über Schrauben befestigt ist. Die Befestigung erfolgt in einem Abstützabschnitt 14, der im Wesentlichen horizontal und waagerecht bei einer üblichen Einstellung des Prothesenfußes 1 herausgerichtet ist. Von dem Anschlussmittel 2 erstreckt sich das Strukturbauteil 10 in einem Bogen 16, der in posteriore Richtung orientiert ist, nach hinten, mit einer konvexen Krümmung, um sich dann in einem Verbindungsabschnitt 18, der konkav ausgebildet ist, in anteriore Richtung zu erstrecken. An den konkaven Verbindungsabschnitt 18 schließt sich ein wiederum konvex geformter Vorderfußabschnitt 20 an, der bis in den Zehenbereich des Prothesenfußes 1 ragt. An dem Zehenbereich ist eine Ausrichteeinrichtung 4 in Gestalt einer Kappe angeklebt, die an der Unterseite ein Sohlenelement 60 aufweist oder an dessen Unterseite ein Sohlenelement 60 ausgebildet ist. Die Ausrichteeinrichtung 4 dient einerseits zur Aufnahme und Befestigung des Vorderfußabschnittes 20 des Strukturbauteils 10, dessen Schutz an dem vorderen Ende sowie an den Seitenkanten sowie zu einer Ausrichtung im Verhältnis zu einem Führungselement 30, das unterhalb des Vorderfußabschnittes 20 beabstandet dazu an der Ausrichteeinrichtung festgelegt ist. Die Art und Weise der Befestigung sowie die Ausgestaltung der Ausrichteinrichtung 4 wird im Detail später erläutert.

Das Führungselement 30 erstreckt sich im Wesentlichen waagerecht in die posteriore Richtung, wobei das Führungselement 30 als eine Blattfeder ausgebildet ist, die von vorne nach hinten verlaufend einen konvexen, konkaven und dann wieder konvexen Konturverlauf aufweist. An dem posterioren Ende des Führungselementes 30 ist ein Formschlusselement 5 in Gestalt einer Fersenkappe angeordnet, an dessen Unterseite ebenfalls eine Sohlenstruktur 60 angeordnet oder ausgebildet ist. Das Formschlusselement 5 kann an dem Führungselement 30 formschlüssig festgelegt sein, beispielsweise durch Aufstecken, Aufklipsen oder Verschrauben, alternativ oder ergänzend kann eine Verklebung und/oder Verschweißung mit dem Führungselement 30 ausgeführt werden.

An der Unterseite des Strukturbauteils 10, im Bereich des Verbindungsabschnittes 18, ist ein zweites Formschlusselement 6 angeordnet, vorteilhafterweise angeklebt, angeschweißt oder durch Formschlusselemente wie Schrauben oder dergleichen daran befestigt. Das zweite Formschlusselement 6 dient zusammen mit dem ersten Formschlusselement 5 zu einer reversiblen Aufnahme eines Feder-Dämpfer-Systems in Gestalt eines Schaumstoffkörpers oder eines Elastomerelementes, das zwischen das Strukturbauteil 10 und das Führungselement 30 eingesetzt wird. Dazu wird das Führungselement 30 von dem Strukturbauteil 10 wegbewegt, in den sich vergrößernden Zwischenraum zwischen der Unterseite des Strukturbauteils 10 und der Oberseite des Führungselementes 30 wird das Feder-Dämpfer-System 40 eingesetzt, nach hinten verschoben und beispielsweise über Vorsprünge und Nute formschlüssig zwischen den beiden federnden Komponenten des Prothesenfußes 10 gehalten.

Der Figur 1 ist bereits zu entnehmen, dass das Strukturbauteil 10 einteilig ausgebildet und als Blattfeder, vorteilhafterweise aus einem faserverstärkten Kunststoffkomposit ausgestaltet ist und der Bogen 16, der eine konvex nach außen gewölbte Form aufweist, in posteriore Richtung über das hintere Ende des Führungselementes 30 hinausragt. Das Feder-Dämpfer-System 40 ist dabei so bemessen, dass der über das hintere Ende des Führungselementes 30 hinausragende Teil des Strukturbauteils 10 oberhalb oder im Bereich eines natürlichen Knöchels verläuft, so dass der Prothesenfuß 1 ohne Weiteres in einen Schuh eingesetzt werden kann.

Durch die in posteriore Richtung vergrößerte Federlänge ist es möglich, bei einem gleichzeitig flachen Prothesenfuß eine vergrößerte Gesamtfederlänge bereitzustellen, so dass eine größere Energiemenge gespeichert und ein tieferes Einsinken bei einem Fersenstoß erfolgen kann, ohne dass die Materialstärke des Strukturbauteils 10 übermäßig vergrößert werden muss, worunter zwangsweise entweder die Haltbarkeit oder die Einstellbarkeit und Elastizität unter einem Fersenstoß leiden würde.

Das Führungselement 30 ist wesentlich dünner als das Strukturbauteil 10, um eine leichte Verformbarkeit zu gewährleisten, wenn der Fersenstoß erfolgt. Dadurch ist es möglich, dass das Führungselement 30 leicht um eine senkrecht zur Laufrichtung und in der Ebene des Führungselementes verlaufende Achse verschwenkt werden kann, so dass nur ein geringer Energieübertrag bzw. eine geringe Krafteinleitung bei einem Fersenstoß über das Führungselement 30 auf den Vorderfußbereich 20 erfolgt. Eine Verschwenkung um eine Hochachse oder um eine in Längserstreckung des Prothesenfußes 1 orientierte Achse ist aufgrund der Struktur des Führungselementes als Blattfeder nicht oder nur sehr eingeschränkt möglich, so dass eine Supinationsbewegung oder Pronationsbewegung des Führungselementes 30 beim Einfedern bzw. Komprimieren des Feder-Dämpfer-Systems vermieden wird.

Das an dem hinteren Ende des Führungselementes 30 angeordnete Formschlusselement 5, auch Fersenkappe genannt, kann einen Absatz ausbilden, so dass an dem hinteren Ende des Führungselementes 30 ein vergrößerter Freiraum zu dem Boden vorhanden ist. Dieser Absatz kann durch das Sohlenelement 60 ausgebildet sein, das aus einem komprimierbaren, elastischen Material bestehen kann, um beim Fersenstoß eine weiche Krafteinleitung in das Führungselement 30 zu bewirken.

Der Vorderfußabschnitt 20 ist als Blattfeder ausgebildet und erlaubt eine Verformung unter Vorfußbelastung. Das Führungselement 30 ist in der Sagittalebene momentenarm an dem Strukturbauteil 10 angebunden. Das Sohlenelement 60 ist dabei so ausgeführt, dass durch eine Belastung im Fersenbereich neben einer Komprimierung des Feder-Dämpfer-Systems 40 eine sich zu der Belastung passende Verlagerung der resultierenden Bodenreaktionskraft einstellt, so dass der Krafteinleitungspunkt möglichst gleichmäßig während der Abrollbewegung entlang der Längserstreckung des Prothesenfußes 1 wandert. Beim Überrollen von der Ferse auf den Vorfuß findet eine Übertragung der in der nach dem initialen Fersenstoß in dem Feder-Dämpfer-System 40 gespeicherten Energie auf den Vorderfußabschnitt 20 dadurch statt, dass nicht allein die Vorfußfeder komprimiert wird, sondern auch das Feder-Dämpfer-System 40 entspannt wird, die darin gespeicherte Energie freigibt und dadurch eine verzögernde Wirkung durch Verformung des Vorfußabschnittes 20 des Vorfußes reduziert wird.

An dem Strukturbauteil 10 ist ein Überlastanschlag 70 vorgesehen, der den Einfederweg des Führungselementes 30 begrenzt, beispielsweise bei Spitzenbelastungen in Sondersituationen, z.B. bei einem Sprung von einer Erhöhung oder dergleichen. Der Überlastanschlag 70 ist als ein Vorsprung des oberen Formschlusselementes 6 ausgebildet, so dass bei einer übermäßigen Belastung der Überlastanschlag 70 mit dem unteren Formschlusselement 5 in direkten Kontakt treten kann und eine weitere Verformung und Komprimierung des Feder-Dämpfer-Systems 40 verhindert.

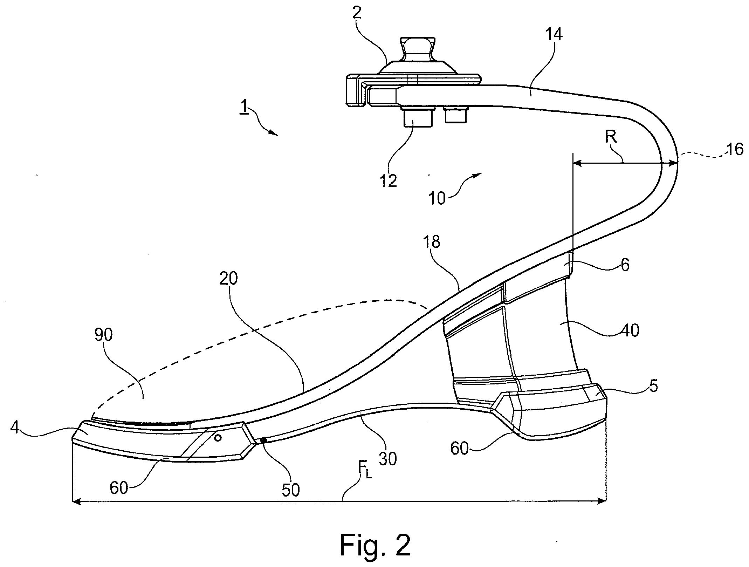

Figur 2 zeigt in einer Seitenansicht die Ausgestaltungsform gemäß Figur 1 mit einem an der Oberseite im Bereich des Vorderfußabschnittes 20 angeordneten Polsters 90, das reversibel an der Oberseite festlegbar ist, beispielsweise über einen Klettverschluss oder dergleichen. In der Seitenansicht ist zu erkennen, dass der Vorderfußabschnitt 20 sich bis zum Fußspitzenbereich des Prothesenfußes 1 erstreckt und an der Oberseite der Ausrichteeinrichtung 4 aufliegt. Die Seitenkanten des Strukturbauteils 10 werden von Seitenwänden, die nach oben von der Ausrichteeinrichtung 4 abragen, zumindest teilweise abgedeckt, so dass der Schutz der empfindlichen Seitenkanten bei faserverstärkten Blattfedern ebenso gegeben ist wie der Schutz von umgebenden Materialien vor den Kanten der Blattfeder. Darüber hinaus kann die Polsterung 90 einen zusätzlichen Schutz bieten.

Innerhalb der Ausrichteeinrichtung 4 ist eine nicht dargestellte Einführöffnung vorgesehen, in die das Führungselement 30, das ebenfalls in Gestalt einer Blattfeder ausgebildet ist, eingeführt wird. Das Führungselement 30 ragt nahezu vollständig in die Ausrichteeinrichtung 4 hinein, lediglich ein kleiner Spitzenbereich dient als vorderer Abschluss und zum Schutz gegen Stoßbelastungen. Die Ausrichteeinrichtung 4 ist bevorzugt aus einem elastischen Material ausgebildet und wird dauerhaft sowohl mit dem Strukturbauteil 10 als auch mit dem Führungselement 30 verklebt. Dazu ist innerhalb der Ausrichteeinrichtung zumindest ein Hohlraum angeordnet, der mit Klebstoff ausgefüllt wird. An der Unterseite der Ausrichteeinrichtung 4 kann ein vorderes Sohlenelement 60 angeordnet oder ausgebildet sein, um ein leichtes Abrollen analog zum Sohlenelement 60 bei der Fersenkappe 5 zu ermöglichen.

Die Fersenkappe 5 bzw. das Formschlusselement 5 weist ebenfalls einen Aufnahmeschlitz für das Führungselement 30 auf, das Führungselement 30 ist in den Schlitz eingeführt und entweder über Formschlusselemente oder über Verkleben oder Verschweißen innerhalb des Formschlusselementes 5 gehalten.

In der Seitenansicht ist zu erkennen, dass das Führungselement 30 eine von vorne nach hinten geschwungene Form mit einer zunächst konvexen, dann konkaven und dann wieder konvexen Formgestaltung aufweist. Dadurch wird die Gesamtfederlänge vergrößert und eine Abrollbewegung sowohl beim Fersenstoß als auch beim der terminalen Standphase erleichtert. Der konkave Bereich des Führungselementes 30 ist im Mittelfußbereich des Prothesenfußes 1 angeordnet und modelliert das natürliche Fußgewölbe nach.

Weiterhin ist zu erkennen, dass das obere Formschlusselement 60 und damit auch die obere Kontaktfläche des Feder-Dämpfer-Systems 40 mit dem Verbindungsabschnitt an der Unterseite des Strukturbauteils 10 ungefähr in einer Ebene mit dem Rückwärtigen Ende des Führungselementes 20 liegt. Der posterior über das Formschlusselement 6 und auch über das posteriore Ende des Führungselementes 30 hinaus stehende Bogen 16 weist einen Überstand R auf, der ungefähr ein Achtel der Gesamtfußlänge FL beträgt, also der gesamten Länge von der Fußspitze bis zum posterioren Ende der Fersenkappe 5. Durch den nach hinten orientierten Überstand R wird die effektive Federlänge des Prothesenfußes 1 massiv vergrößert, so dass eine größere Energiespeicherfähigkeit bei zugleich größerem Einfederweg und gleichzeitig schlanker Federgestaltung erreicht werden kann, da die gespeicherte Energie über die vergrößerte Federlänge und nicht über eine Verdickung des Materials in der Blattfeder des Strukturbauteils 10 erfolgt. Je dünner eine Blattfeder aus einem Faserverbundwerkstoff ausgestaltet ist, desto haltbarer ist sie, da innerhalb der Blattfeder geringere Scherkräfte auftreten.

Die Kontaktfläche des Feder-Dämpfer-Systems 40 mit dem Strukturbauteil 10 erfolgt vorteilhafterweise im Bereich des Verbindungsabschnittes 18, also in dem Bereich, in dem die konvexe Gestalt des Bogens 16 in die konkave Formgebung des Verbindungsabschnittes 18 über geht. Das Feder-Dämpfer-System 40 aus einem Schaumstoff oder Elastomerelement leitet beim initialen Fersenstoß die Kräfte, die beim Auftreten entstehen, direkt in Richtung des Anschlussadapters 2 aufgrund der Orientierung des Feder-Dämpfer-Elementes in Richtung auf den Anschlussadapter 2. Aufgrund der Einleitung der Kräfte in den Verbindungsbereich findet ein Einfedern während des Fersenstoßes über den sich proximal anschließenden Abschnitt des Strukturbauteils 10 statt, also in dem Bogen 16 und dem Anschlussabschnitt 14, so dass eine Kraftübertragung und Energiespeicherung in dem Vorderfußabschnitt 20 nicht stattfindet. Der Vorderfußabschnitt 20 wird also bei einem Fersenstoß nicht negativ aufgeladen, da keine Krafteinleitung in den Vorderfußabschnitt 20 erfolgt. Aufgrund der geringen Materialstärke des Führungselementes 30 und der vereinfachten Biegung um eine fiktive Schwenkachse 50 senkrecht zu der Blattebene wird auch über das Führungselement 30 kein Energieeintrag in den Vorderfußabschnitt 20 erfolgen. Bei einer Ausgestaltung des Feder-Dämpfersystems 40 aus einem Schaum oder einem entsprechenden Elastomerelement, treten keine Scherkräfte innerhalb des Feder-Dämpfer-Elementes auf, so dass auch keine Zugkräfte in dem Führungselement 30 eingeleitet werden.

Das Feder-Dämpferelement 40, das auswechselbar ausgestaltet sein kann, weist vorteilhafterweise ein progressives Feder-Dämpfer-Verhalten auf und kann beispielsweise als ein Zwei-Komponenten-Schaumstoff, ein Zwei-Komponenten-Elastomer oder eine Kombination mehrerer Materialien und/oder mehrerer Dichten gleicher Materialien aufweisen, um die gewünschten Feder-Dämpfer-Eigenschaften zu gewährleisten. Das Feder-Dämpfer-Element 40 ist formschlüssig in den Formschlusselementen 5, 6 gehalten, Hinterschnitte sind in den Formschlusselementen 5, 6 vorgesehen, die in Ausnehmungen oder Nuten in dem Feder-Dämpfer-System 40 eingreifen.

In der Seitenansicht der Figur 2 ist weiterhin zu erkennen, dass das Strukturbauteil 10 als einteilige Blattfeder als gelenkloser Prothesenfuß 1 ausgebildet ist und im Knöchelbereich eine Federverlängerung aufweist, um die effektive Federlänge zu vergrößern. Aufgrund der vergrößerten effektiven Federlänge ist eine vertikale Verformung des Prothesenfußes 1 erleichtert möglich, wobei der Scheitelpunkt des Bogens 16 über die natürliche Gesamtfußlänge FL hinaus steht, also über das rückwärtige Ende des als Basisfeder ausgebildeten Führungselementes 30. Der Prothesenfuß 1 ist unmittelbar in einen Schuh einführbar und bedarf keiner Fußkosmetik. Der Prothesenfuß 1 hat aufgrund seiner Ausgestaltung ein hohes Verformungsvermögen, eine große Energiespeicherkapazität während der Standphase, stellt eine hohe Haltbarkeit aufgrund der vergleichsweise geringen Materialstärke der Blattfederkomponenten zur Verfügung und ermöglicht eine geringe Prothesenfußhöhe. Darüber hinaus stellt die Ausführung eine Fersenkomponente bereit, so dass es möglich ist, den Prothesenfuß 1 auch während normaler Aktivitäten zu tragen. Darüber hinaus ist der Prothesenfuß 1 für sportliche Aktivitäten wie Joggen oder dergleichen bevorzugt geeignet. Aus dem Stand der Technik bekannte Sportfüße sind in der Regel nicht in einem Schuh zu tragen und sind für das Stehen in der Regel nicht geeignet. Übliche gelenklose Prothesenfüße für den täglichen Einsatz können zwar in einer Fußkosmetik oder gegebenenfalls sogar in einem Schuh unmittelbar eingesetzt werden, ermöglichen aber nicht das hohe Maß an Verformbarkeit, wie es der Prothesenfuß 1 der vorliegenden Erfindung ermöglicht.

In der Seitenansicht gemäß Figur 2 ist an dem Anschlussmittel 2 die zunächst im Wesentlichen waagerechte Blattfeder im Bereich des Anschlussabschnittes 14 zu erkennen, von denen sich in hintere, posteriore Richtung ein leicht nach unten gekippter Verlauf fortsetzt. Grundsätzlich ist es auch möglich, dass ein weiterer waagerechter Verlauf oder ein leicht nach oben geneigter Verlauf sich anschließt. An den Anschlussabschnitt 14 schließt sich der hintere Bogen 16 an, der sich über ca. ein Achtel der Gesamtfußlänge FL hinter dem Fersenende des Prothesenfußes 1 befindet und eine konkave Krümmung aufweist. Nach Ende der konkaven Krümmung geht das Strukturbauteil 10 in eine konvexe Krümmung mit einem im Vergleich zum Bogen 16 größeren Radius über und von dort aus wieder in eine konkave Krümmung mit einem ebenfalls größeren Krümmungsradius als der Bogen 16. Beim Fersenauftritt werden das Feder-Dämpfer-System 40 und der Federabschnitt mit dem Bogen 16 und dem Anschlussabschnitt 14 in Reihe geschaltet, durch den hinteren Bogen 16 ist nach einer Verformung des Strukturbauteils 10 in Gestalt einer Verlagerung des Anschlussmittels 2 in Richtung auf den Boden möglich. Dazu ragt der Bogen 16 über die Kraftlinie, die sich von dem Fersenauftritt durch das Feder-Dämpfer-System zum Anschlussmittel 2 erstreckt nach hinten hinaus. Je größer der rückwärtige Überstand R ist, desto weicher wird der Prothesenfuß 1, der maximale Überstand R ergibt sich aus dem Einsatzzweck, den verwendeten Materialien und den Vorlieben der Prothesenfußnutzer.

Beim Überrollen zur mittleren Standphase wird das Feder-Dämpfer-System 40 teilweise entlastet, da nun der Vorfußabschnitt 20 und das vordere Sohlenelement 60 den Boden berührt. Dadurch beginnt sich das gesamte Strukturbauteil 10 zu verformen. Der bisher noch nicht verformte Vorderfußabschnitt 20 wird gebogen, die vertikal während der mittleren Standphase eingebrachte Kraft wird durch ein weiteres Verformen im Bereich des Bogens 16 und des Anschlussabschnittes 14 in potenzielle Energie verwandelt. Dadurch wird die vertikale Stoßbelastung reduziert. Bei einem weiteren Überrollen auf den Vorfuß wird das Feder-Dämpfer-System 40 komplett entlastet und das Strukturbauteil 10 trägt die komplette Last. Aufgrund des Bogens 16 erfolgt die beim Vorderfußabheben statt in der Rückgabe zunächst vorrangig vertikal und dreht sich im letzten Drittel der Entlastung in Richtung der Laufrichtung. Der Scheitelpunkt des Bogens liegt ungefähr im Bereich des natürlichen Knöchels und ist in posteriorer Richtung über die natürliche Knöchelposition verlagert. Die Fersensteifigkeit wird vorrangig durch das Feder-Dämpfer-System, den Bogen 16 und den Anschlussabschnitt 14 des Strukturbauteils 10 gesteuert. Der Kraftangriffspunkt wird durch die Formgebung und des Sohlenelementes 60 möglichst lange nahe bei der Ferse gehalten, um das Verformungsverhalten der Ferse angenehm für den Nutzer zu gestalten. Durch die schmale Ausgestaltung des Führungselementes 30 wird wenig Kraft auf den Vorderfußabschnitt 20 aufgebracht, dies wird durch die doppelt gekrümmte Formgebung des Fersenelementes weiter verringert.

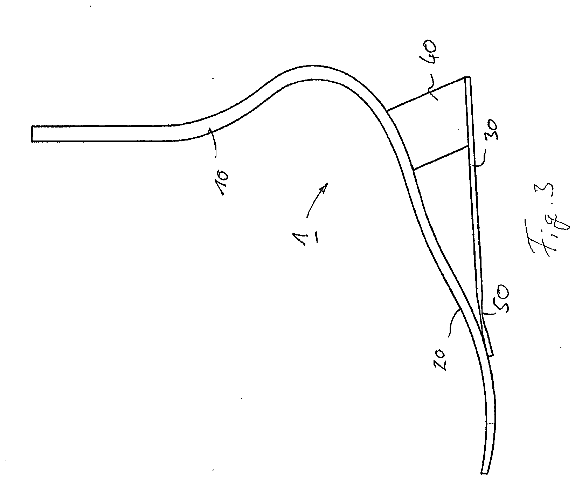

In der Figur 3 ist eine weitere Variante des Prothesenfußes 1 dargestellt. Im dargestellten Ausführungsbeispiel ist der Prothesenfuß 1 mit einer einteiligen Kombination der Stützstruktur 10 und dem Vorderfußelement 20 ausgestattet. An dem proximalen Ende der Stützstruktur 10 erfolgt dann eine Befestigung an einem Unterschenkelschaft oder anderen Befestigungselementen. Das plattenartige, im Wesentlichen ebene Führungselement 30 ist an der dem Boden zugewandten Unterseite im Ballenbereich des Vorderfußelementes 20 befestigt, beispielsweise anlaminiert, angeschweißt, angeschraubt oder angeklebt. Die Achse 50, die als Schwenkachse ausgebildet ist, wird durch ein Filmscharnier ausgebildet, das eine Verschwenkung um eine innerhalb des Firmscharniers liegende Achse ermöglicht, ein Verdrehen um eine Längsachse in anterior-posterior-Richtung sowie um eine Achse in proximal-distal-Richtung wird jedoch aufgrund der Steifigkeit des Führungselementes 30 weitgehend verhindert. Das Feder-Dämpfer-System ist unmittelbar an der Stützstruktur 10 in der Nähe des Knöchels befestigt.

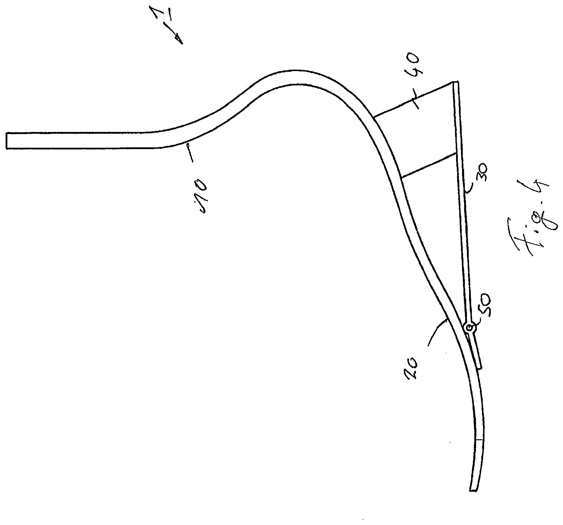

Eine Variante der Figur 3 ist in der Figur 4 dargestellt, bei der statt eines Filmscharniers ein freies Gelenk mit einer Achse 50 vorgesehen ist, das als Klappe ausgebildet sein kann.

Allen Ausführungsformen ist gemeinsam, dass ein fersenseitiges Feder-Dämpfer-System 40 über das Führungselement 30 in der Sagittalebene momentenarm mit weiteren Elementen der Fußstruktur verbunden ist, also entweder mit dem Vorderfußabschnitt 20 oder dem Strukturbauteil 10. Kräfte außerhalb der Wirkrichtung des Feder-Dämpfer-Elementes 40 werden in dem Führungselement 30 aufgenommen und über das Gelenk, das die Verschwenkung um die Achse 50 zulässt, abgeleitet. Das Feder-Dämpfer-System 40 ist auf seiner Oberseite knöchelnah an dem Strukturbauteil 10 abgestützt, die Führungselemente 30 sind im Wesentlichen als ebene Struktur ausgebildet und stützen das an der Unterseite des Strukturbauteils abgestützte Feder-Dämpfer-System 40 auf der Unterseite ab. Bei einer außermittigen Krafteinleitung auf die Ferse kann die ebene Struktur des Führungselementes 30 jedoch tordieren, so dass die Kontaktfläche vergrößert wird. Die bei Fersenbelastung eingeleiteten Horizontalkräfte, die in Laufrichtung oder senkrecht zur Laufrichtung wirken, werden von der Struktur des Führungselementes 30 aufgenommen und über das Gelenk, das als Schwenkachse 50 ausgebildet ist, in die knöchelnahe Stützstruktur eingeleitet. Die gelenkige Befestigung des Führungselementes 30 stützt damit das fersenseitige Feder-Dämpfer-System gegen Scherung bei Horizontalkräften ab. Die gelenkige Lagerung ist dabei so ausgeführt, dass es durch seine Breite gut in der Lage ist, die auftretenden Scherkräfte aufzunehmen. Es können auch mehrere Lagerungsstellen auf einer gemeinsamen Achse 50 nebeneinander angeordnet sein, um eine Hintereinanderanordnung von Gelenken zu realisieren.

Prothesenfüße sollen die Kraft während des Auftrittsstoßes abmildern, eine ausreichende Stabilität beim Überrollen vermitteln und beim Abstoßen diejenige Energiemenge zurückgeben, die der Nutzer beim Gehen gut beherrschen kann. Die hierzu erforderliche Verformung der einzelnen Komponenten bringt die Leistungsfähigkeit der eingesetzten Hochleistungswerkstoffe an deren Grenzen. Daher werden in Prothesenfüße aus Hochleistungswerkstoffen oft Federsysteme eingesetzt, deren Elemente nahezu starr miteinander verbunden sind. Diese Elemente schützen sich gegenseitig durch die gekoppelte Wirkung vor Überlastung, lassen aber andererseits keine unabhängige Wirkung auf unterschiedliche Lasteinleitung, z. B. von Ferse und Vorderfuß zu.

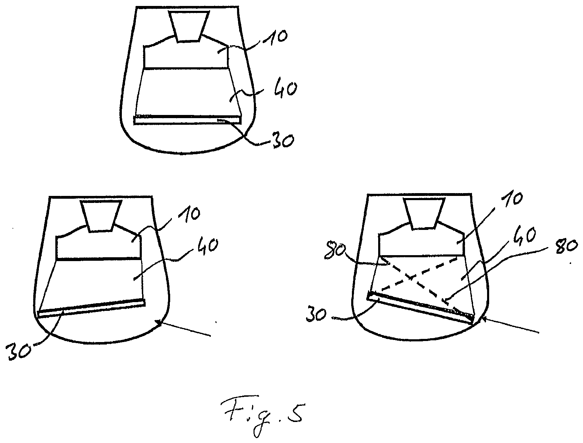

In der Figur 5 sind in einer Rückansicht das Strukturbauteil 10 und das Feder-Dämpfer-Element 40 sowie das Führungselement 30 gezeigt. In der oberen Darstellung ist ein unbelasteter Prothesenfuß dargestellt, es ist zu erkennen, dass das Führungselement 30 über das fersenseitige Feder-Dämpfer-System 40 mit dem Strukturbauteil 10 gekoppelt ist. Eine Kraftübertragung von dem Führungselement 30 auf die Strukturbauteil 10 soll nur innerhalb der Wirkrichtung des Feder-Dämpfer-Systems 40 erfolgen, bei einem Fersenauftritt ist dies die Kraftrichtung innerhalb der Sagittalebene und innerhalb der Medialebene von distal nach proximal. Kräfte außerhalb der Wirkrichtung des Feder-Dämpfer-Systems 40 werden über das Führungselement 30 aufgenommen und über die quasi-gelenkige Lagerung abgeleitet, entweder in den Vorderfußabschnitt 20 und darüber in das Strukturbauteil 10 oder direkt in das Strukturbauteil 10. Die sohlenseitige Struktur des Führungselementes 30 zur Führung des Feder-Dämpfer-Systems 40 ist distal so ausgeführt, dass durch die Belastung der Ferse neben einer Komprimierung des Feder-Dämpfer-Systems 40 eine sich zu der Belastung passende Verlagerung der resultierenden Bodenreaktionskraft beispielsweise durch Torsion des Führungselementes 30 einstellt. Eine solche Situation ist in der linken unteren Darstellung der Figur 5 gezeigt. Eine Seitenkraft wirkt auf das Führungselement 30 ein, was zu einer Verlagerung des Führungselementes 30 führt.

In der rechten unteren Darstellung der Figur 5 ist ein Ausführungsbeispiel mit zwei Stabilisierungselementen 80 in Gestalt von Zugelementen vorgesehen, die kreuzweise angeordnet sind. Die Lagerung des Führungselementes 30 ist dabei so ausgeführt und wird durch die Stabilisierungselemente 80 dergestalt gestützt, dass eine seitliche Kraft nicht nur abgestützt wird, sondern dass der seitlich angreifenden Kraft durch eine Schwenkbewegung durch eine Verlagerung des Kraftangriffspunktes auf die Seite der Krafteinwirkung entgegengewirkt wird.

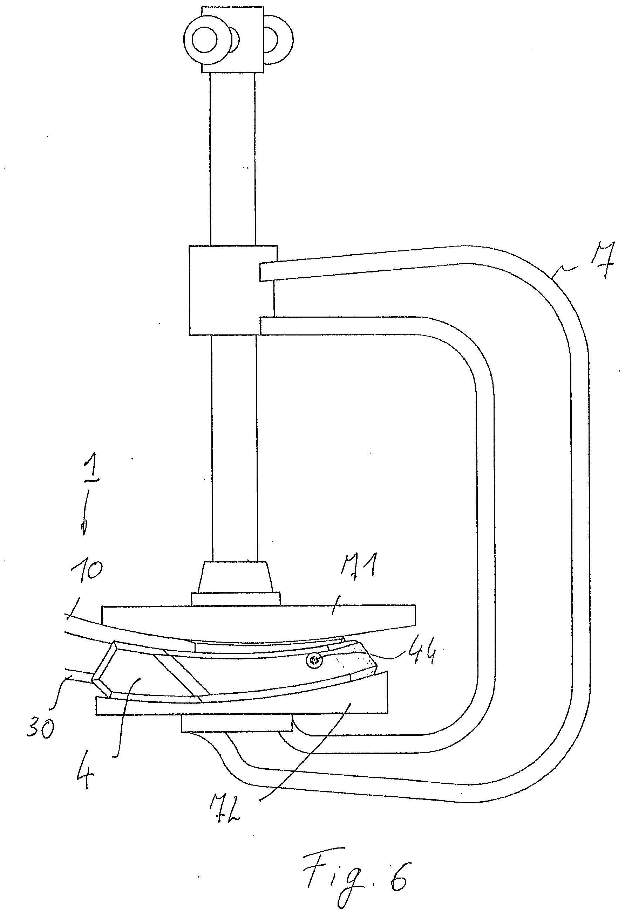

Figur 6 zeigt in einer Seitenansicht eine schematische Darstellung eines vorderen Teils eines Prothesenfußes 1. Der Prothesenfuß 1 weist zwei Strukturbauteile 10, 30 auf, die als Blattfedern aus einem faserverstärkten Kunststoff hergestellt sind. Es ist der Vorderfußbereich des Prothesenfußes 1 dargestellt, das erste Strukturbauteil 10 ist eine Vorderfußfeder, das zweite Strukturbauteil 30 das Führungselement. Die Vorderfußfeder 10 erstreckt sich schräg nach oben zu einem oberen Anschlusspunkt, an dem Befestigungseinrichtungen oder Anschlussmittel zur Befestigung an einem Unterschenkelrohr oder einem Unterschenkelschaft befestigbar sind. Das Führungselement 30, auch Basisfeder genannt führt bis in den Fersenbereich, wobei sich eine Fersenfeder von der Basisfeder 30 zur Vorfußfeder 10 und/oder den oberen Anschlussmitteln erstrecken kann.

Das Strukturbauteil 10 und das Führungselment 30 sind einer Ausrichteeinrichtung 4 zugeordnet, die als Kunststoffspritzgussteil ausgebildet ist. Die Ausrichteeinrichtung 4 kann aus einem Polyurethan, einem technischen Polyethylen, einem technischen Polyurethan, Gummi oder einem anderen Kunststoff, vorzugsweise Elastomer, bestehen. Die Ausrichteeinrichtung 4 weist einen Einführschlitz für das Führungselement 30 sowie einen Aufnahmebereich an der Oberseite für das erste Strukturbauteil 10 auf, auf den das erste Strukturbauteil 10 aufgelegt werden kann. Der Auflagebereich ist von Wänden umrahmt, so dass das erste Strukturbauteil 10 in einer definierten Position zu der Ausrichteeinrichtung 4 aufgelegt werden kann, wenn sich die Kontur des Strukturbauteils 10 an die Wände um den Auflagebereich herum anlegen.

Das Führungselement 30 wird in einen nicht dargestellten Schlitz innerhalb der Ausrichteeinrichtung eingeführt, so dass die Unterseite des Führungselementes 30 oder der Blattfeder von einer geschlossenen Oberfläche der Unterseite der Ausrichteeinrichtung 4 abgedeckt ist. Zwischen dem Strukturbauteil 10 und dem Führungselement 30 ist ein Abstandshalter ausgebildet, der die beiden Komponenten beabstandet zueinander festhält. Durch das Einführen des Führungselementes 30 in die Ausrichteeinrichtung 4 ist auch diese Komponente definiert zugeordnet, beispielsweise indem es in einem Schlitz oder in einer Nut innerhalb der Aufnahmeeinrichtung 4 geführt ist. Dadurch bilden die beiden Komponenten 10, 30 und die Aufnahmeeinrichtung 4 einen Hohlraum aus, der im Wesentlichen geschlossen ist. In einer Seitenwand der Ausrichteeinrichtung 4 ist ein Zuführanschluss 44 vorgesehen, der in strömungstechnischer Verbindung mit dem nicht dargestellten Hohlraum steht und durch den Klebstoff in den Hohlraum eingeführt bzw. hineingepumpt werden kann. Auf der dem Zuführanschluss 44 abgewandten Seite ist ein Auslasskanal vorgesehen, der ebenfalls in strömungstechnischer Verbindung mit dem Hohlraum steht, so dass die innerhalb des Hohlraumes befindliche Luft austreten kann und der Hohlraum vollständig mit Klebstoff befüllt werden kann.

Die Komponenten 10, 30 und die Ausrichteeinrichtung 4 sind in einer Presse 7, die als übliche Schraubzwinge ausgebildet sein kann, gehalten. An der Presse 7 sind zwei Pressenschuhe 71, 72 angeordnet, die eine Kontur aufweisen, die der jeweilig zugeordneten Kontur der orthopädietechnischen Komponente 1 entspricht. Im dargestellten Ausführungsbeispiel ist der obere Pressenschuh 71 mit einer konvexen Wölbung und der untere Pressenschuh 72 mit einer konkaven Wölbung ausgestattet, so dass einerseits die Unterseite der Aufnahmeeinrichtung 4 und andererseits die Oberseite des ersten Strukturbauteils 10 vollflächig an der Oberfläche des jeweiligen Pressenschuhs 71, 72 anliegen können. Wird die Presse 7 geschlossen und Druck auf die Pressenschuhe 71, 72 ausgeübt, wird das erste Strukturbauteil 10 auf die Oberfläche der Auflagefläche an der Ausrichteeinrichtung 4 gepresst, so dass der zwischen dem Strukturbauteil 10 und dem Führungselement 30 oben und unten und an den Seitenflächen durch die Ausrichteeinrichtung 4 gebildete Hohlraum abgeschlossen ist und eine Zufuhr von Klebstoff nur durch den Zuführanschluss 44 und ein Austreten von Luft und gegebenenfalls überschüssigen Klebstoff durch den Auslasskanal erfolgen kann.

Nach dem Einführen des Klebstoffes wird der Pressdruck aufrechterhalten, bis der Klebstoff ausgehärtet ist, so dass eine dauerhafte Verbindung zwischen dem ersten Strukturbauteil 10, dem Führungselement 30 und der Ausrichteeinrichtung 4 erzielt wird. Nach Aushärtung des Klebstoffes verbleibt die Ausrichteeinrichtung 4 an der orthopädietechnischen Komponente 1 und dient einerseits als Schutz für das Strukturbauteil 10 und das Führungselement 30 und andererseits als funktionale Komponente der orthopädietechnischen Komponente, beispielsweise als Formgebung für den Prothesenfuß, als Polster, als Sohlenstruktur oder bei anderen Ausgestaltungsformen als Aufnahmeeinrichtung oder Schutzeinrichtung für weitere Komponenten.

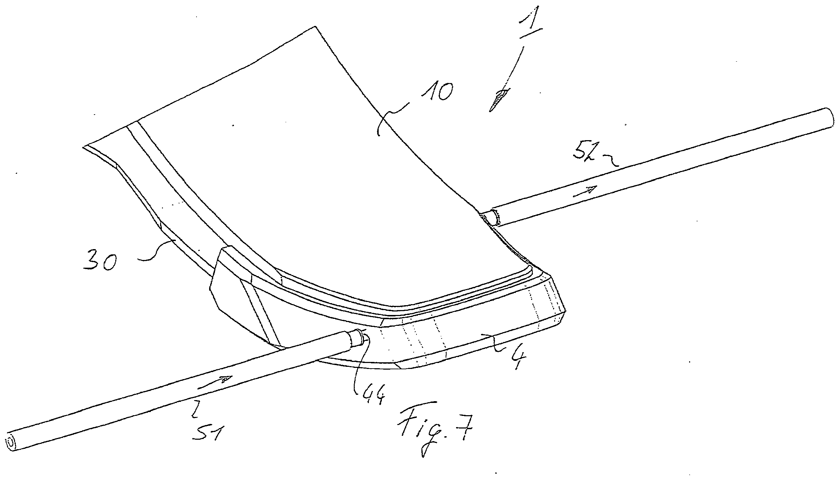

Figur 7 zeigt in einer perspektivischen Schrägdraufsicht die Fertigung der orthopädietechnischen Komponente 1, zumindest das Verbinden des Strukturbauteils 10 und des Führungselementes 30 mit der Ausrichteeinrichtung 4. An der Ausrichteeinrichtung 4 ist an dem Zuführanschluss 44 eine Zuführeinrichtung 51 angebracht, die im dargestellten Ausführungsbeispiel als Schlauch oder Rohr ausgebildet ist, und durch die Klebstoff, wie durch den Pfeil angedeutet, in den nicht dargestellten Hohlraum eingeleitet wird. Der Hohlraum ist an der Oberseite und an der Unterseite durch das Strukturbauteil 10 und das Führungselement 30, an der Vorderseite und an den Seitenkanten durch die Seitenwände der Ausrichteeinrichtung 4 und an der Rückseite zwischen den Blattfedern 10, 30 durch einen Abstandshalter, der dichtend sowohl an der Unterseite des ersten Strukturbauteils 10 als auch an der Oberseite des Führungselementes 30 anliegt, gebildet und abgeschlossen. In der Figur 7 ist die Presse 7 nicht dargestellt, während der Zufuhr des Klebstoffes wird jedoch die Zuordnung der jeweiligen Komponenten 4, 10, 30 durch die Presse 7 oder eine andere, geeignete Fixiereinrichtung aufrechterhalten.

Klebstoff wird durch die Zuführeinrichtung 51 und den Zuführanschluss 44 in den Hohlraum eingeführt, die in dem Hohlraum befindliche Luft wird durch den Klebstoff verdrängt und durch eine Auslasseinrichtung 52 abtransportiert. Die Auslasseinrichtung 52 ist an einem nicht dargestellten Auslasskanal, der strömungstechnisch in Verbindung mit dem Hohlraum innerhalb der Aufnahmeeinrichtung 4 steht, verbunden, so dass Luft und gegebenenfalls überschüssiger Klebstoff aus dem Auslasskanal durch die Auslassöffnung 51 austreten kann, wie durch den Pfeil angedeutet. Sowohl der Zuführanschluss 44 als auch der Auslasskanal sind bevorzugt in einem Abstandshalter angeordnet, der sicherstellt, dass die Blattfedern 10, 30 zueinander beabstandet gehalten werden. Dadurch wird sichergestellt, dass durch die Anordnung der Blattfedern 10, 30 an oder in der Ausrichteeinrichtung 4 nicht durch die Zuordnung der Blattfedern 10, 30 zueinander versperrt werden.

Die nicht dargestellte Presse 7 hält die Zuordnung der Komponenten 4, 10, 20 zueinander, solange der Klebstoff aushärtet. Nach dem Aushärten werden die Zuführeinrichtung 51 und die Auslasseinrichtung 52 von der Ausrichteeinrichtung 4 getrennt, beispielsweise abgeknickt, so dass ein nahezu glatter Abschluss der Ausrichteeinrichtung 4 im Bereich des Zuführanschlusses 44 und des Auslasskanals erreicht werden kann. Dies kann beispielsweise durch eine Sollbruchstelle an der Zuführeinrichtung 51 und/oder der Auslasseinrichtung 52 im Bereich des Anschlusses an die Ausrichteeinrichtung 4 gewährleistet werden.

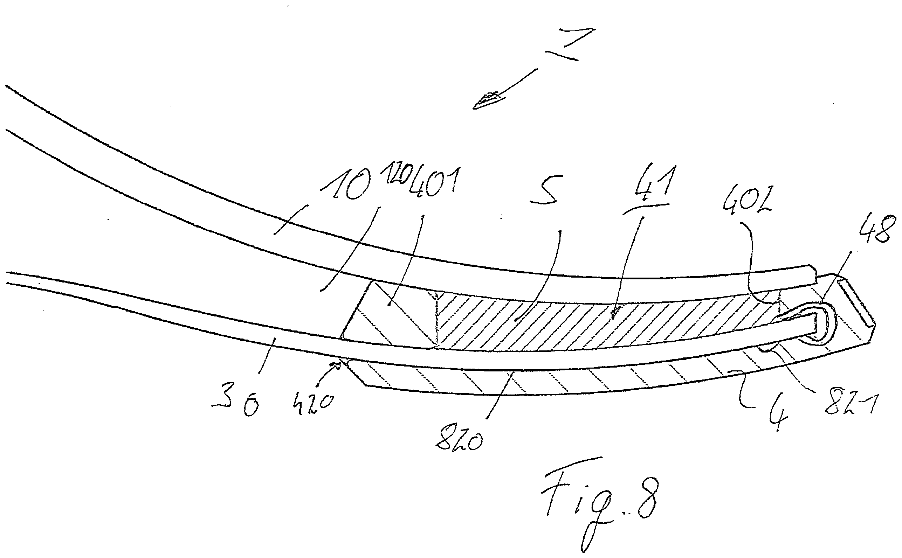

Figur 8 zeigt eine Schnittdarstellung durch den vorderen Teil eines fertig montierten Prothesenfußes 1 mit einem oberen, auf der Ausrichteeinrichtung 4 aufliegenden ersten Strukturbauteil 10 in Gestalt einer Vorderfußfeder aus einem faserverstärkten Kunststoffmaterial, der Ausrichteeinrichtung 4 und dem in die Ausrichteeinrichtung 4 eingeführten Führungselement 30 in Gestalt einer Basisfeder, die ebenfalls als Blattfeder aus einem faserverstärkten Kunststoffmaterial ausgebildet ist. Die obere Blattfeder liegt auf einer oberen Auflagefläche auf, die untere Blattfeder auf einer unteren Auflagefläche 820. An dem vorderen, im dargestellten Ausführungsbeispiel rechten Ende der Ausrichteeinrichtung 4 ist ein Kanal 48 ausgebildet, der von der Unterseite des Führungselementes 30 zu dem Hohlraum 41 führt, der von dem Führungselement 30, dem ersten Strukturbauteil 10 sowie der Ausrichteeinrichtung 4 eingeschlossen wird. In der Auflagefläche 820, die durch die dem Führungselement 30 zugewandte Oberfläche der Basis der Ausrichteeinrichtung 4 gebildet ist, sind Vertiefungen 821 ausgebildet, so dass auch unterhalb des Führungselementes 30 aufgrund einer strukturierten Oberfläche oder der Vertiefungen 821, die in strömungstechnischer Verbindung mit dem Hohlraum 41 stehen, Klebstoff 5, der den Hohlraum 41 vollständig ausfüllt, in die Vertiefungen 821 eindringen kann, so dass zumindest das untere Führungselement 30 an mehreren Seiten oder an mehreren Stellen von dem Klebstoff 5 umgeben ist. Vorteilhafterweise ist ein Zuführanschluss 44 an der geodätisch tiefsten Stelle der Ausrichteeinrichtung 4 während der Montage angeordnet, in der dargestellten Ausrichtung, beispielsweise an der Unterseite der Ausrichteeinrichtung 4, und steht sowohl mit den Vertiefungen 821 und, aufgrund des Kanals 48, auch mit dem Hohlraum 41 in strömungstechnischer Verbindung. Wird nun an der tiefsten Stelle Klebstoff 5 zugeführt, drückt sich dieser durch die strukturierte Oberfläche auf der Oberseite der Basis der Ausrichteeinrichtung 4 durch die Vertiefungen 821, durch den Kanal 48 in den Hohlraum 41, wobei die bislang darin eingeschlossene Luft durch den nicht dargestellten Auslasskanal abgeführt wird.

Der Figur 8 ist zudem eine Einführöffnung 420 für das Führungselement 30 zu entnehmen, die im dargestellten Ausführungsbeispiel als Schlitz ausgebildet ist und auf der Höhe der Oberseite der Basis, die die Auflagefläche 820 bildet, endet. Oberhalb der Einführöffnung 420 ist ein erster Abstandshalter 490 angeordnet, auf den das erste Strukturbauteil 10 aufgelegt ist, so dass sich zwischen dem ersten Strukturbauteil 10 und dem Führungselement 30 ein Zwischenraum 120 bildet, der sich auch nach vorne hin fortsetzt, da an dem vorderen Ende ein zweiter Abstandshalter 402 ausgebildet ist, der als Auflagefläche für das erste Strukturbauteil 10 dient. Der Figur 8 ist zu entnehmen, dass die Einführöffnung 420 so bemessen ist, dass die untere Blattfeder dicht anliegend hindurchgedrückt und eingeschoben werden kann. Dadurch wird vermieden, dass beim Einführen des Klebstoffes 5 Klebstoff aus einem Bereich der Einführöffnung 420 um das Führungselement 30 herum austreten kann. Die Dichtwirkung wird durch das Anpressen des ersten Strukturbauteils 10 auf den Abstandshalter 401 und damit auf das Führungselement 30 vergrößert. Aufgrund des zweiten Pressenschuhs 72 liegt die Auflagefläche 820 dicht an dem Führungselement 30 an, so dass kein Klebstoff beim Befüllen des Hohlraums 51 entweichen kann.

Das vordere Ende des Führungselementes 30 ist vollständig in der Aufnahmeeinrichtung 4 aufgenommen und wird allseitig geschützt und umgeben, die Einfassung oder Umrandung der oberen Auflagefläche für das erste Strukturbauteil schützt die Blattfeder an dem Umfang, an der Unterseite findet der Schutz durch den Klebstoff sowie die Auflagefläche auf der Ausrichteeinrichtung 4 statt, lediglich die Oberseite ist ungeschützt.

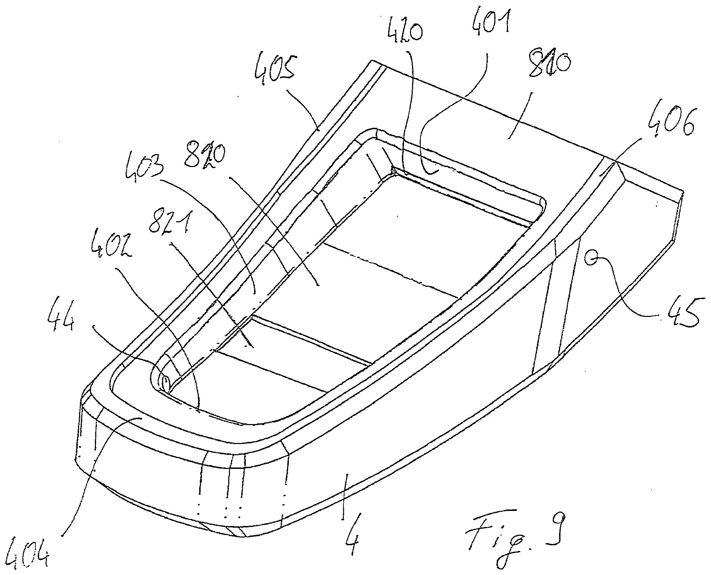

Figur 9 zeigt in einer perspektivischen Darstellung einer Aufnahmeeinrichtung 4 gemäß der Ausführungsform der vorherigen Figuren. Neben dem Zuführanschluss 44, dem Auslasskanal 45 und der unteren Auflagefläche 820 ist die Vertiefung 821 etwas vergrößert dargestellt. Der Kanal 48, der in strömungstechnischer Verbindung mit der Vertiefung 821 steht, ist nicht dargestellt. Es sind die Abstandshalter 401, 402 an der Rückseite und der Vorderseite zu erkennen. Die Abstandshalter 401, 402 bilden an ihren Oberseiten gleichzeitig eine obere Auflagefläche 810 für das nicht dargestellte erste Strukturbauteil aus, das mit seiner Unterseite auf die Auflagefläche 810 gepresst wird. Der Einführschlitz oder die Einführöffnung 420 endet auf der Höhe der unteren Auflagefläche 820. Seitlich neben der Auflagefläche 820 ist in den seitlichen Abstandshaltern 403 eine Nut eingearbeitet, in die das blattförmige Führungselement 30 eingeführt wird, bis es an den vorderen Abschluss der Ausrichteeinrichtung 4 anschlägt.

Die obere Auflagefläche 810 wird von Seitenwänden 404, 405, 406 eingefasst, die in ihrer Materialstärke der des oberen Strukturbauteils 10 entsprechen können. Durch die Seitenwände 404, 405, 406 wird eine definierte Zuordnung des oberen Strukturbauteils 10 zu der Ausrichteeinrichtung 4 und damit zu dem unteren Führungselement 30 gewährleistet, wenn die vorderen und seitlichen Kanten des Strukturbauteils 10 an den jeweiligen Seitenwänden 404, 405, 406 anliegen. Entspricht die Höhe der Seitenwände 404, 405, 406 der Materialstärke des oberen Strukturbauteils 10, kann ein bündiger Abschluss der Oberflächen erreicht werden.

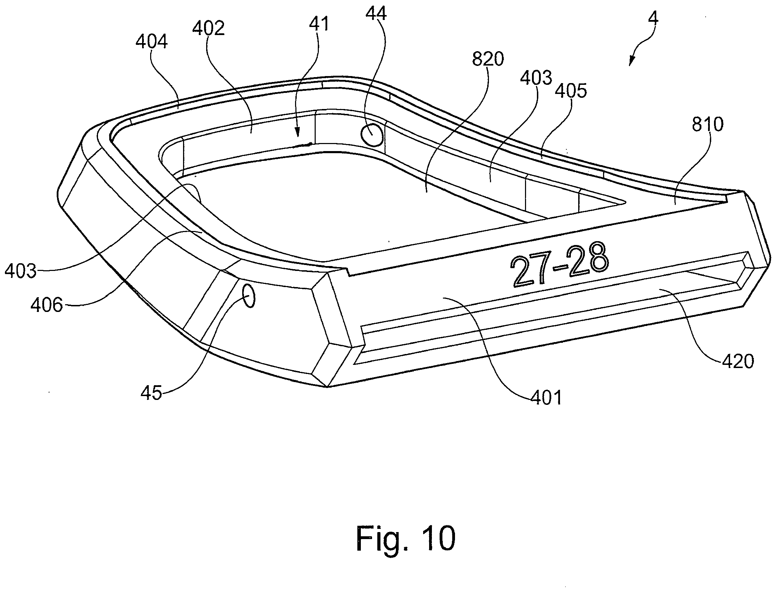

Figur 10 zeigt die Ausrichteeinrichtung 4 gemäß den vorherigen Ausführungsformen in einer Ansicht von schräg hinten, aus der der hintere Abstandshalter 401, der vordere Abstandshalter 402 sowie die Einführöffnung 420 sehr gut zu erkennen ist. Ebenso ist zu erkennen, dass der Zuführanschluss 44 tiefer als der Auslasskanal 45 ausgebildet ist, wobei sowohl der Zuführanschluss 44 als auch der Auslasskanal 45 innerhalb der Abstandshalter 403 ausgebildet sind. Unterhalb der Abstandshalter 403 ausgebildeten Seitenwände ist durch einen Hinterschnitt eine Nut ausgebildet, in der das Führungselement 30 einführbar ist. Die erhöhten Seitenwände 404, 405, 406, die über die obere Auflagefläche 810 hinaus stehen, sind ebenso zu erkennen, wie die im dargestellten Ausführungsbeispiel ebene Auflagefläche 820 auf der Oberseite der Basis der Ausrichteeinrichtung 4. Durch die Seitenwände 402, 403 und den hinteren Abstandshalter 401 wird innerhalb der Ausrichteeinrichtung 4 eine Aufnahme gebildet, die vollständig mit Klebstoff verfüllt werden kann. Durch das Einführen des unteren Führungselementes 30 durch die Einführöffnung 420 wird die Einführöffnung 420 geschlossen, so dass die Aufnahme nach dem Einführen des Führungselementes 30 nur oben offen ist. Wird das nicht dargestellte Strukturbauteil 10 auf die obere Auflagefläche 810 aufgelegt, ist der Hohlraum 41 geschlossen. Nach Befüllen des Hohlraums 41 mit dem Klebstoff steht dieser in klebender Verbindung sowohl mit der Ausrichteeinrichtung 4 als auch mit den beiden Blattfedern 10, 30.

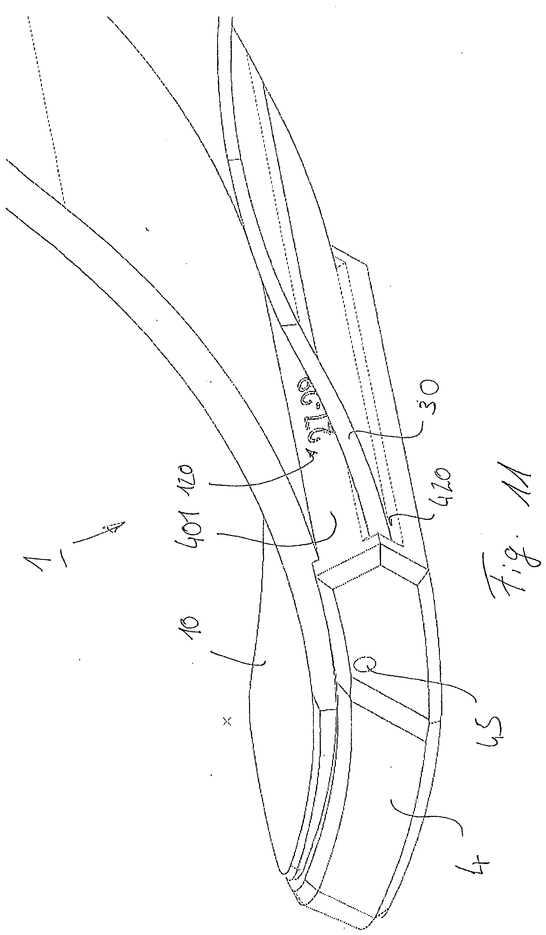

Figur 11 zeigt einen vorderen Teil der orthopädietechnischen Komponente in Gestalt eines Prothesenfußes 1 von schräg hinten in einem fertig montierten Zustand. Das Führungselement 30 ist in die Einführöffnung 420 eingeführt, das obere Strukturbauteil 10 ist unter Ausbildung eines Zwischenraumes 12, der durch den Abstandshalter 401 sichergestellt ist, auf der nicht dargestellten Auflagefläche 810 aufgelegt und gehalten. Der Auslasskanal ist in einer Seitenwand angeordnet, die Komponenten 4, 10, 30 sind dauerhaft über den Klebstoff innerhalb der Aufnahmeeinrichtung 4 verbunden.

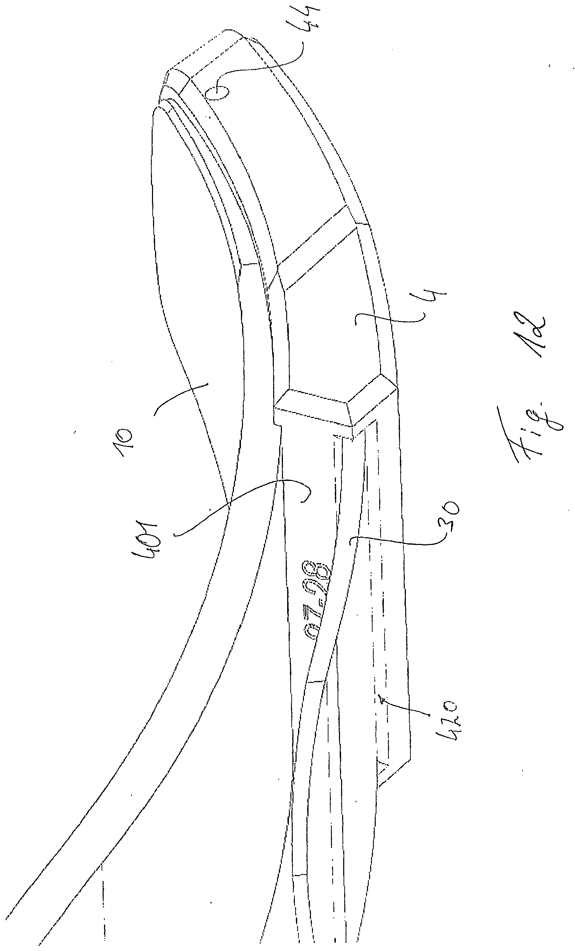

Figur 12 zeigt die Ausführungsform gemäß Figur 11 von der anderen Seite, der Zuführanschluss 44 ist an einer vorderen Seitenwand der Ausrichteeinrichtung 4 angeordnet.

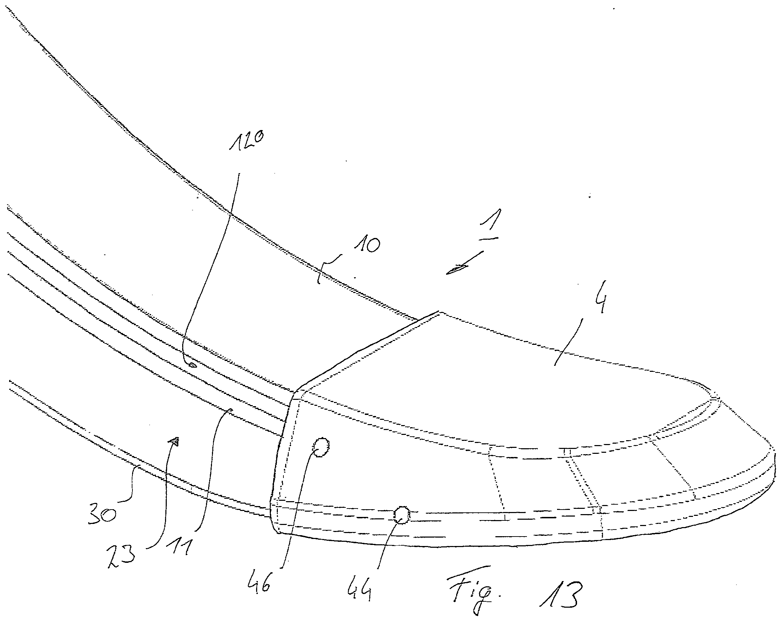

Figur 13 zeigt eine Variante der Erfindung, bei der statt lediglich zweier Strukturbauteile, wie dies in den Figuren 6 bis 12 dargestellt ist, drei Strukturbauteile 10, 11, 30 miteinander über eine Ausrichteeinrichtung 4 verbunden sind. Die orthopädietechnische Komponente 1 ist wiederum als ein Prothesenfuß ausgebildet und weist eine Basisfeder als Führungselement 30 auf. Die Vorfußfeder ist als eine parallel geschaltete, doppelte Blattfederanordnung ausgebildet, die zwei Blattfedern als mittleres Strukturbauteil 11 und oberes Strukturbauteil 10 aufweisen. Die Orientierung der Doppelfeder und der Basisfeder entspricht der Orientierung, wie sie weiter oben beschrieben wurde, grundsätzlich sind auch abweichende Orientierungen und Ausrichtungen der Komponenten 10, 11, 30 zueinander möglich und vorgesehen.

Zwischen dem unteren Führungselement 30 und dem zweiten, mittleren Strukturbauteil 11 ist ein Zwischenraum 23 ausgebildet, während zwischen dem ersten, oberen Strukturbauteil 10 und dem mittleren Strukturbauteil 11 ein zweiter Zwischenraum 120 ausgebildet ist. Der Zwischenraum wird durch entsprechende Abstandshalter innerhalb der Ausrichteeinrichtung 4 ausgebildet.

Die Ausrichteeinrichtung 4 ist im Gegensatz zu der vorherigen Ausführungsform oben geschlossen, das heißt, dass das obere Strukturbauteil 10 nicht auf eine obere Auflagefläche aufgelegt wird, um einen Hohlraum abzuschließen, vielmehr werden alle Strukturbauteile und das Führungselement 30 von der rückwärtigen Seite durch Einführöffnungen in die Ausrichteeinrichtung 4 eingeschoben.

Da sich die Beabstandung der jeweiligen Strukturbauteile 10, 11 und des Führungselementes 30 innerhalb der Ausrichteeinrichtung 4 fortsetzt, werden zumindest zwei Hohlräume innerhalb der Ausrichteeinrichtung 4 gebildet und voneinander getrennt, so dass im dargestellten Ausführungsbeispiel zwei Zuführanschlüsse 44, 46 vorgesehen sind, so dass eine getrennte Befüllung der Hohlräume erfolgen kann. Hierdurch ist es möglich, beispielsweise unterschiedliche Klebstoffe, unterschiedliche Klebstofftemperaturen oder andere Prozessmerkmale zu realisieren, wenn diese prozesstechnisch erforderlich ist.

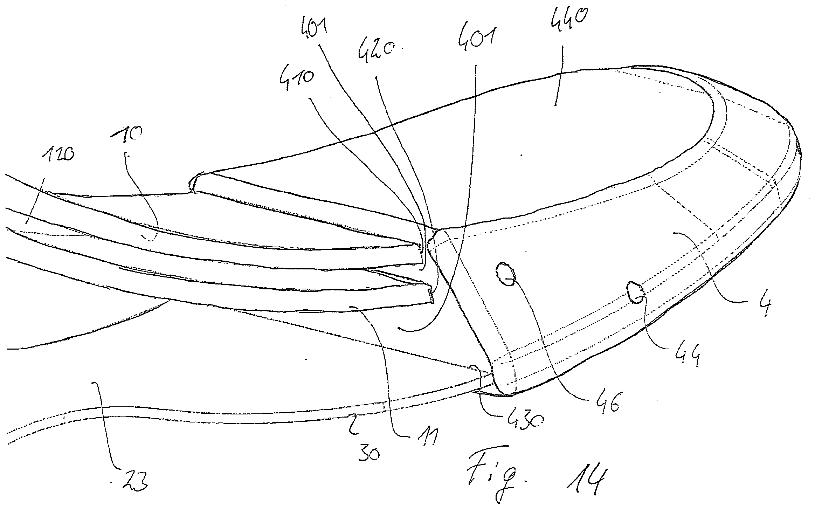

Figur 14 zeigt die Ausführungsform gemäß Figur 13 in einer Ansicht von schräg hinten, Die drei Einführöffnungen 410, 420, 430 an der rückwärtigen Stirnfläche der Ausrichteeinrichtung 4 sind ebenso zu erkennen wie die beiden Zuführanschlüsse 44, 46 und die durch die rückwärtige Wand ausgebildeten hinteren Abstandshalter 401 zwischen den Blattfedern 10, 11, 30.

Die Einführöffnung 430 für das Führungselement 30 liegt, wie in der vorherigen Ausführungsform, auf dem Niveau der unteren Auflagefläche 820, auch die Nut, bevorzugt eine umlaufende Nut in der Seitenwand und eine gegebenenfalls vorhandene Strukturierung der Auflagefläche können vorgesehen sein. Statt der oben offenen Ausführung ist in dem dargestellten Ausführungsbeispiel gemäß Figur 14 eine Abdeckung 440 vorgesehen, so dass auch die Oberseite des oberen Strukturbauteils 10 von dem Material der Ausrichteeinrichtung 4 abdeckt wird. Somit sind die vorderen Enden der Strukturbauteile 10, 20 und des Führungselementes 30 vollständig von der Ausrichteinrichtung 4 umgeben und über den Klebstoff miteinander und mit der Ausrichteeinrichtung 4 verbunden.

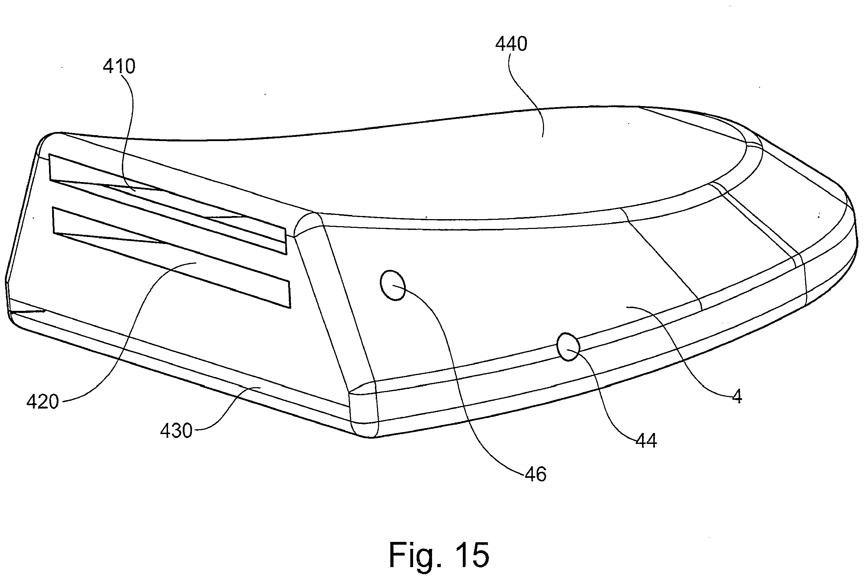

Figur 15 zeigt die Ausrichteeinrichtung 4 gemäß dem zweiten Ausführungsbeispiel in einer Einzeldarstellung. Es sind die drei Einführöffnungen 410, 420, 430 an der Rückseite ebenso zu erkennen, wie die beiden seitlichen Zuführanschlüsse 44, 46, die einen Zugang zu den Zwischenräumen bzw. durch das Einführen der Blattfedern 10, 11, 30 entstehenden Hohlräume innerhalb der Ausrichteeinrichtung 4 zu erkennen. Die obere Abdeckung 440 bildet den oberen Abschluss, die Basis der Ausrichteeinrichtung 4 bildet den unteren Abschluss und eine Art Sohle bei einer Ausgestaltung der orthopädietechnischen Komponente als Prothesenfuß.

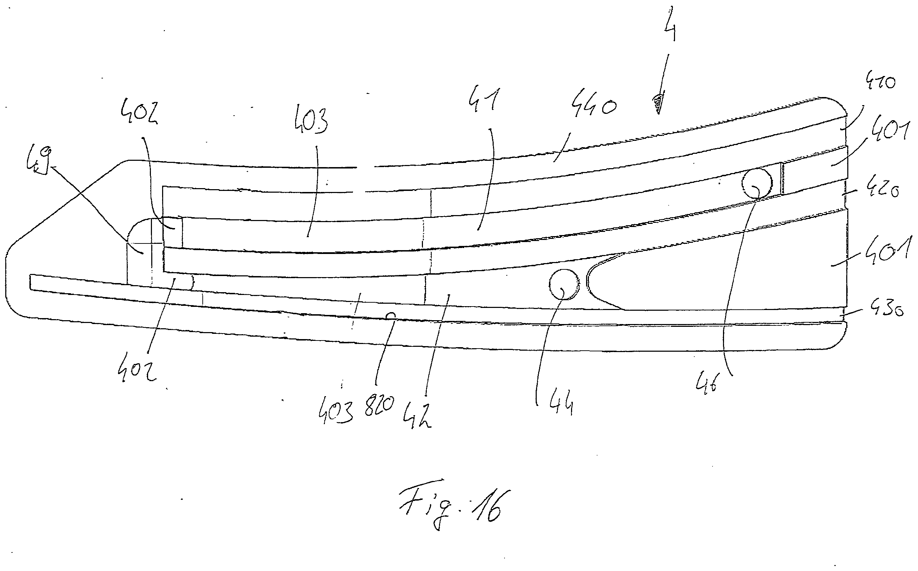

Figur 16 zeigt eine Schnittansicht der Ausrichteeinrichtung 4, aus der die Einführöffnungen 410, 420, 430, die hinteren Abstandshalter 401 sowie die vorderen und seitlichen Abstandshalter 402, 403 zu erkennen sind. Ebenso ist ein Kanal durch die vorderen Abstandshalter 402 hindurchgehend ausgebildet, so dass Klebstoff, wenn er durch die Zuführanschlüsse 44, 46 seitlich in die Hohlräume 41, 42 durch die Ausrichteeinrichtung 4 und die darin aufgenommenen Strukturbauteile ausgebildet werden, eingelassen werden. Alternativ zu der in den Figuren 13 bis 15 dargestellten Ausführungsform ist es möglich, das nur die untere Öffnung 44 als Zuführanschluss ausgebildet ist, während die obere Öffnung als Auslasskanal ausgebildet ist, so dass Klebstoff durch den Zuführanschluss 44, durch den Hohlraum 42 und den Kanal 49 in den Hohlraum 41 gelangt und dann durch den Auslasskanal austritt. Die Auflagefläche 820 kann strukturiert sein und ebenfalls von Klebstoff umspült oder benetzt werden, so dass das mittlere Strukturbauteil sowohl auf der Unterseite als auch auf der Oberseite von Klebstoff umgeben ist und beidseitig mit je einer anderen Blattfeder darauf verbunden ist. Die geschlossene Abdeckung 440 ist ebenso zu erkennen, wie die geschlossene vordere Spitze, eine Einführnut für das untere Strukturbauteil, die über den Kanal 49 in vordere Richtung hinausragt. Die Zuführanschlüsse 44, 46 bzw. der Zuführanschluss 44 und der Auslasskanal sind in den seitlichen Abstandshaltern 403 ausgebildet.

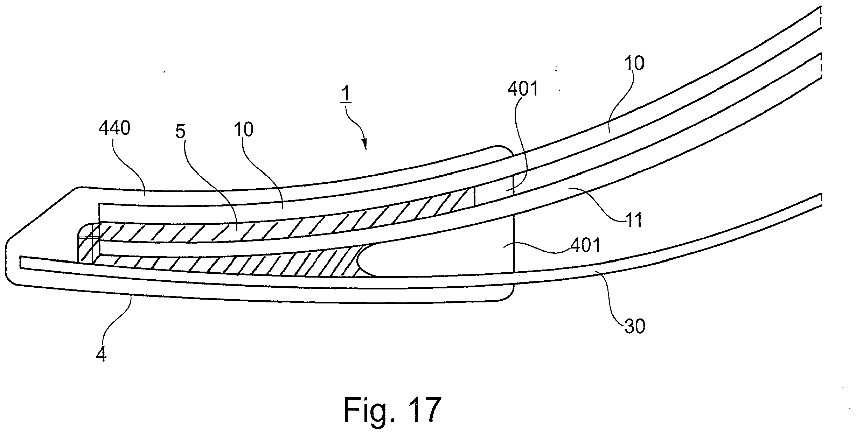

Figur 17 zeigt das vordere Ende der orthopädietechnischen Komponente 1 im montierten Zustand in einer schematischen Schnittdarstellung. Die zwei Strukturbauteile 10, 11 und das Führungselement 30 in Gestalt von Blattfedern sind durch die jeweiligen Einführöffnungen in die Ausrichteeinrichtung 4 eingeführt und die hinteren Abstandshalter 401, die nicht dargestellten Abstandshalter 403 und die vorderen Abstandshalter 402 zueinander beabstandet in der Ausrichteinrichtung 4 gehalten. Der Klebstoff 5 ist durch den nicht dargestellten Zuführanschluss 44 in den Hohlraum 41 eingeführt, durch den Kanal 49 in den oberen Hohlraum 42 eingedrungen und durch den nicht dargestellten oberen Auslasskanal 45 abgeführt wurden. Durch den dichtenden Abschluss der Einführöffnungen 410, 420, 430 um die Blattfedern 10, 11, 30 herum ist kein Klebstoff 5 während der Fertigung rückwärtig ausgetreten. Der Klebstoff umgibt das zweite Strukturbauteil 11 an der Oberseite, an der Vorderseite sowie an der Unterseite.

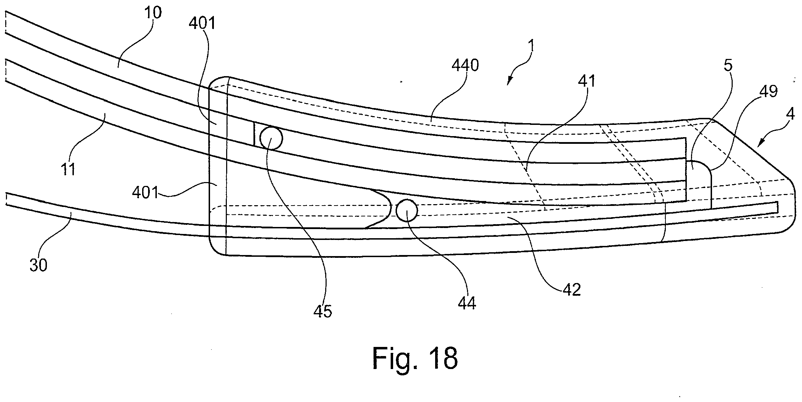

Figur 18 zeigt eine Seitenansicht des montierten Prothesenfußes bzw. der orthopädietechnischen Komponente 1, bei der statt zweier Zuführanschlüsse ein unterer Zuführanschluss 44 und ein oberer Auslasskanal 45 in der Ausrichteeinrichtung 4 vorgesehen sind. Die drei eingeführten Strukturkomponenten 10, 11, 30 sind ebenso zu erkennen, wie die hinteren Abstandshalter 401, die Zwischenräume oder Hohlräume 41, 42, die nach hinten durch die eingeführten Strukturbauteile 10, 20, 30 abgedichtet werden und die obere Abdeckung 440, durch die auch die obere Blattfeder oder das obere Strukturbauteil 10 vollständig von der Ausrichteinrichtung 4 abgedeckt und geschützt wird.

Der Klebstoff wird durch den Zuführanschluss 44 in den unteren Hohlraum 42 durch den Kanal 49 in den oberen Hohlraum 41 und durch den Auslasskanal 45 hinausgedrückt, sobald Klebstoff aus dem oben gelegenen Auslasskanal 45 austritt, wird die Zufuhr des Klebstoffes durch den Zuführanschluss 44 gestoppt, die Bauteile 10, 11, 30 in der gewünschten Zuordnung gehalten und gewartet, bis der Klebstoff ausgehärtet ist, so dass sämtliche Komponenten 10, 11, 30, 4 dauerhaft miteinander verbunden sind.

Eine weitere Variante der Erfindung umfasst die Ausgestaltung, bei der die Ausrichteeinrichtung 4 ohne untenseitigen Boden ausgebildet ist. Die Ausrichteeinrichtung 4 ist hier als ein Rahmen mit Auflageflächen für die oben und unten aufgelegten Strukturbauteile 10, bzw. Führungselemente 30 ausgebildet. Der Rahmen ist umlaufend mit einer eingeschlossenen Öffnung, die durch die Blattfedern 10, 30 zu einem Hohlraum komplettiert wird, in den Klebstoff 5 eingeführt wird, so dass die Unterseite des oberen Strukturbauteils 10 und die Oberseite des Führungselementes 30 einander gegenüberliegend mit Klebstoff 5 benetzt und miteinander an der Ausrichteeinrichtung 4 verklebt werden. Beide Blattfedern 10, 30 werden auf die jeweilige Auflagefläche gedrückt und gedrückt gehalten, bis der Klebstoff 5 ausgehärtet ist, durch das Andrücken auf die Auflageflächen wird der gebildete Hohlraum abgedichtet, überschüssiger Klebstoff 5 tritt nur durch den in einem Abstandshalter angeordneten Auslasskanal, vorzugsweise über eine Auslasseinrichtung aus, so dass das Bauteil nicht mit Klebstoff 5 verunreinigt wird. Der Aufbau entspricht ansonsten dem der Figur 8

Durch das auch als zu Erfindung gehörende, oben beschriebene Verfahren und die erfindungsgemäße orthopädietechnische Komponente ist es möglich, zwei Strukturbauteile wie Blattfedern, insbesondere zwei Faserverbundwerkstoffe mit einem flüssigen Klebstoff zu verkleben und diese Strukturbauteile gleichzeitig zu umgeben, um dadurch eine schützende Umhüllung bereitzustellen. Die Ausrichteeinrichtung passt an oder auf die zu verbindenden Komponenten und bildet eine Kavität zwischen ihnen aus, die den Aufnahmeraum für den flüssigen Klebstoff bildet. Um den Klebstoff in die Kavität oder den Hohlraum einzuführen und gleichzeitig die Kavität zu entlüften, sind vergleichsweise kleine Öffnungen in Gestalt von Zuführkanälen oder Auslasskanälen in die Ausrichteinrichtung oder die Form und Umhüllung integriert. In diese Zuführanschlüsse und Auslasskanäle können Schlauchverbinder eingesetzt werden, die mit einem Zuführschlauch und einem Entlüftungsschlauch verbunden werden können. Um sicherzustellen, dass der Hohlraum oder die Kavität sicher abgedichtet ist, können die Strukturbauteile zusammengepresst oder gegen die Aufnahmeeinrichtung 4 gepresst werden, wobei diese durch flexible Materialien geschehen kann. Vorzugsweise ist das Material der Ausrichteeinrichtung 4 ein flexibles, elastisches Material, so dass eine abdichtende Anlage an den Strukturbauteilen durch Ausüben von Druck in Richtung auf die Strukturbauteile gewährleistet werden kann. Nach dem Einführen des Klebstoffes und dem Aushärten werden die Schlauchverbinder von der Ausrichteeinrichtung oder der Formenhülle entfernt und das Verbindungsverfahren ist abgeschlossen. Die Form dient nun nicht mehr als Begrenzung für den Klebstoff, sondern wird als Hülle oder Umhüllung der Strukturbauteile eingesetzt, um die Strukturbauteile gegen eine Beschädigung zu schützen und darüber hinaus um weitere Bauteile, beispielsweise eine Umhüllung oder eine Kosmetik vor Schäden durch die miteinander verbundenen Strukturbauteile, die scharfkantig sein können, zu schützen.

Durch die Vorrichtung und das Verfahren ist es möglich, dass eine Form für einen flüssigen Klebstoff zum Verbinden zweier Strukturbauteile bereitgestellt wird. Die Ausrichtung der zu verbindenden Komponenten ist durch die Ausrichteinrichtung 4 gewährleistet, ebenso werden die zu verbindenden Bauteile geschützt, das Herstellverfahren ist sauber und eine Nachbearbeitung der Klebestelle ist nicht notwendig. Der Verbrauch von Klebstoff wird eingeschränkt, da kein überschüssiger Klebstoff austreten kann und ein definiertes Volumen durch die jeweiligen Hohlräume als Grundlage für die Berechnung der zugeführten Klebstoffmenge dienen kann. So ist durch ein mengengesteuertes Zuführen von Klebstoff sichergestellt, dass einerseits eine minimale Klebstoffmenge genutzt und andererseits stets ausreichend Klebstoff zum vollständigen Ausfüllen des Hohlraumes bereitgestellt wird.

## Patentansprüche

1. Prothesenfuß mit einem Strukturbauteil (10) mit proximalen Anschlussmitteln (2) zur Befestigung des Prothesenfußes an einem Unterschenkelrohr, Unterschenkelschaft oder einem Prothesenkniegelenk, einem sohlenseitigen Führungselement (30), das ein posteriores Ende aufweist, und einem an dem Strukturbauteil (10) festgelegten oder ausgebildeten Vorderfußabschnitt (20) und einem dem Strukturbauteil (10) zugeordneten, fersenseitigen Feder-Dämpfer-System (40), das bei einem Fersenauftritt komprimiert wird und sich auf dem sohlenseitigen Führungselement (30) abstützt, wobei das Strukturbauteil (10) als eine Blattfeder ausgebildet ist, die sich von den proximalen Anschlussmitteln (2) in posteriore Richtung erstreckt, einen Bogen ausbildet und in anteriore und distale Richtung geführt ist, **dadurch gekennzeichnet, dass** der Bogen posterior über das Führungselement (30) übersteht.

2. Prothesenfuß nach Anspruch 1, **dadurch gekennzeichnet, dass** der Bogen über die natürliche Knöchelposition in posteriorer Richtung hinausragt.

3. Prothesenfuß nach Anspruch 1 oder 2, **dadurch gekennzeichnet, dass** das Führungselement (30) an der Unterseite des Feder-Dämpfer-Systems (40) angeordnet und als Blattfeder im Vorderfußabschnitt (20) fest an dem Strukturbauteil (10) oder um eine quer zur Längserstreckung des Prothesenfußes orientierte Achse (50) frei beweglich an der Stützstruktur (10) gelagert ist.

4. Prothesenfuß nach Anspruch 3, **dadurch gekennzeichnet, dass** das Führungselement (30) als eine Blattfeder ausgebildet ist.

5. Prothesenfuß nach einem der voranstehenden Ansprüche, **dadurch gekennzeichnet, dass** das Feder-Dämpfer-System (40) als Schaumstoffelement oder Elastomerelement ausgebildet ist.

6. Prothesenfuß nach Anspruch 5, **dadurch gekennzeichnet, dass** das Feder-Dämpfer-System (40) reversibel zwischen der Unterseite der Stützstruktur (10) und der Oberseite des Führungselementes (30) angeordnet ist.

7. Prothesenfuß nach einem der voranstehenden Ansprüche, **dadurch gekennzeichnet, dass** an der Unterseite des Führungselementes (30) ein Sohlenelement (60) aus einem elastischen Material angeordnet ist.

8. Prothesenfuß nach einem der voranstehenden Ansprüche, **dadurch gekennzeichnet, dass** der Vorderfußabschnitt (20) als Blattfeder ausgebildet ist oder zumindest eine Blattfeder aufweist.

9. Prothesenfuß nach einem der voranstehenden Ansprüche, **dadurch gekennzeichnet, dass** zwischen dem Führungselement (30) und der Stützstruktur (10) ein Überlastanschlag (70) angeordnet ist.

10. Prothesenfuß nach einem der voranstehenden Ansprüche, **dadurch gekennzeichnet, dass** das Führungselement (30) um eine in anterior-posterior-Richtung verlaufende Achse und/oder um eine in proximal-distal-Richtung verlaufende Achse unverlagerbar gelagert ist.

11. Prothesenfuß nach einem der voranstehenden Ansprüche, **dadurch gekennzeichnet, dass** das posteriore Ende des Führungselementes ein freies Ende darstellt und an dem freien Ende des Führungselementes (30) ein Absatz (35) in Proximalrichtung angeordnet oder als konvexe Wölbung ausgebildet ist.

12. Prothesenfuß nach einem der voranstehenden Ansprüche, **dadurch gekennzeichnet, dass** das Strukturbauteil (10) elastisch und einteilig ausgebildet ist.

13. Prothesenfuß nach einem der voranstehenden Ansprüche, **dadurch gekennzeichnet, dass** an dem posterioren Ende des Führungselementes (30) und der Unterseite des Strukturbauteils (10) je ein Formschlusselement (5, 6) zur Festlegung des Feder-Dämpfer-Systems (40) festgelegt sind.

14. Prothesenfuß nach einem der voranstehenden Ansprüche, **dadurch gekennzeichnet, dass** der Bogen in anterior-posterior-Richtung über das Feder-Dämpfer-System um eine Strecke übersteht, die zwischen 10% und 30%, bevorzugt zwischen 12,5% und 25% der Fußlänge F_{L} beträgt.

15. Prothesenfuß nach einem der voranstehenden Ansprüche, **dadurch gekennzeichnet, dass** an der Oberseite des Strukturbauteils (10) im Vorderfußbereich (20) eine Kontur bildende Polsterung (90) angeordnet ist.

16. Prothesenfuß nach einem der voranstehenden Ansprüche, **dadurch gekennzeichnet, dass** von den proximalen Anschlussmitteln (2) das Strukturbauteil (10) in dem Bogen konkav gekrümmt ist, im Bereich der Abstützung des Feder-Dämpfer-Systems eine konvexe Krümmung mit einem im Vergleich zu dem Bogen größeren Krümmungsradius und daran anschließend eine wiederum konkave Krümmung im Vorderfußabschnitt (20) aufweist.

17. Prothesenfuß nach einem der voranstehenden Ansprüche, **dadurch gekennzeichnet, dass** das Führungselement (30) als eine Blattfeder mit einer von der Ferse bis zur Fußspitze konkav-konvex-konkav verlaufenden Form ausgebildet ist.

18. Prothesenfuß nach einem der voranstehenden Ansprüche, **dadurch gekennzeichnet, dass** der Vorderfußabschnitt (20) und das Führungselement (30) über eine Ausrichteeinrichtung (4) einander zugeordnet sind.

19. Prothesenfuß nach Anspruch 18, **dadurch gekennzeichnet, dass** der Vorderfußabschnitt (20) und das Führungselement (30) und die Ausrichteeinrichtung (4) miteinander verklebt sind.

20. Prothesenfuß nach einem der voranstehenden Ansprüche, **dadurch gekennzeichnet, dass** dieser zur Anordnung in einem Schuh ausgebildet ist.

21. Prothesenfuß nach einem der voranstehenden Ansprüche, **dadurch gekennzeichnet, dass** das Führungselement (30) ein Filmscharnier aufweist.

## Claims

1. A prosthetic foot comprising a structural component (10) with proximal connection means (2) for fastening the prosthetic foot to a below knee tube, below knee shank or a prosthetic knee joint, a sole-side guide element (30) comprising a posterior end, and a forefoot portion (20) fastened to, or embodied at, the structural component (10) and a heel-side spring-damper system (40) assigned to the structural component (10), said spring-damper system being compressed at a heel strike and supporting itself on the sole-side guide element (30), the structural component (10) is embodied as a leaf spring, which extends from the proximal connection means (2) in the posterior direction, forms an arc and is guided in the anterior and distal directions, **characterized in that** the arc projecting beyond the guide element (30) in the posterior direction.

2. The prosthetic foot as claimed in claim 1, **characterized in that** the arc projects beyond the natural ankle position in the posterior direction.

3. The prosthetic foot as claimed in claim 1 or 2, **characterized in that** the guide element (30) is arranged at the lower side of the spring-damper system (40) and, as a leaf spring in the forefoot portion (20), mounted securely at the structural component (10) or at the support structure (10) in a manner freely movable about an axis (50) oriented transversely in relation to the longitudinal extent of the prosthetic foot.

4. The prosthetic foot as claimed in claim 3, **characterized in that** the guide element (30) is embodied as a leaf spring.

5. The prosthetic foot as claimed in one of the preceding claims, **characterized in that** the spring-damper system (40) is embodied as a foam-material element or an elastomeric element.

6. The prosthetic foot as claimed in claim 5, **characterized in that** the spring-damper system (40) is reversibly arranged between the lower side of the support structure (10) and the upper side of the guide element (30).

7. The prosthetic foot as claimed in one of the preceding claims, **characterized in that** a sole element (60) made of an elastic material is arranged at the lower side of the guide element (30).

8. The prosthetic foot as claimed in one of the preceding claims, **characterized in that** the forefoot portion (20) is embodied as a leaf spring or comprises at least one leaf spring.

9. The prosthetic foot as claimed in one of the preceding claims, **characterized in that** an overload stop (70) is arranged between the guide element (30) and the support structure (10).

10. The prosthetic foot as claimed in one of the preceding claims, **characterized in that** the guide element (30) is mounted in a non-displaceable manner about an axis extending in the anterior-posterior direction and/or about an axis extending in the proximal-distal direction.

11. The prosthetic foot as claimed in one of the preceding claims, **characterized in that** the posterior end of the guide element establishes a free end and that a heel (35) is arranged at the free end of the guide element (30) in the proximal direction or embodied as a convex arch.

12. The prosthetic foot as claimed in one of the preceding claims, **characterized in that** the structural component (10) has an elastic and monolithic design.

13. The prosthetic foot as claimed in one of the preceding claims, **characterized in that** a form-fit element (5, 6) for fixing the spring-damper system (40) is fixed in each case at the posterior end of the guide element (30) and at the lower side of the structural component (10).

14. The prosthetic foot as claimed in one of the preceding claims, **characterized in that** the arc extends beyond the spring-damper system in the anterior-posterior direction by a section which is between 10% and 30%, preferably between 12.5% and 25%, of the foot length F_{L}.

15. The prosthetic foot as claimed in one of the preceding claims, **characterized in that** contour-forming cushioning (90) is arranged at the top side of the structural component (10) in the forefoot region (20).

16. The prosthetic foot as claimed in one of the preceding claims, **characterized in that**, from the proximal connection means (2), the structural component (10) is concavely curved in the arc, has a convex curvature with, compared to the arc in the region of the support of the spring-damper system, a larger radius of curvature and, following thereon, curvature which is once again concave in the forefoot portion (20).

17. The prosthetic foot as claimed in one of the preceding claims, **characterized in that** the guide element (30) is embodied as a leaf spring with a form with a concave-convex-concave profile from the heel to the tip of the foot.

18. The prosthetic foot as claimed in one of the preceding claims, **characterized in that** the forefoot portion (20) and the guide element (30) are assigned to one another by way of an alignment device (4).

19. The prosthetic foot as claimed in claim 18, **characterized in that** the forefoot section (20) and the guide element (30) and the alignment device (4) are adhesively bonded to one another.

20. The prosthetic foot as claimed in one of the preceding claims, **characterized in that** it is embodied for arrangement in a shoe.

21. The prosthetic foot as claimed in one of the preceding claims, **characterized in that** the guide element (30) has a film hinge.

## Revendications

1. Prothèse de pied, comportant un composant de structure (10) ayant des moyens de raccordement proximaux (2) pour fixer la prothèse de pied sur un tube de bas de jambe, sur une emboîture de bas de jambe ou sur une prothèse d'articulation de genou, un élément de guidage (30) côté plante qui présente une extrémité postérieure, et une partie d'avant-pied (20) fixée ou réalisée sur le composant de structure (10) et un système amortisseur élastique (40) côté talon qui est associé au composant de structure (10) et qui, lors d'une pose du talon, est comprimé et s'appuie sur l'élément de guidage (30) côté plante, le composant de structure (10) étant réalisé sous la forme d'un ressort à lame qui s'étend depuis les moyens de raccordement proximaux (2) en direction postérieure, qui forme un arc et qui est guidé en direction antérieure et distale,'
**caractérisée en ce que**
l'arc dépasse postérieurement au-delà de l'élément de guidage (30).

2. Prothèse de pied selon la revendication 1,
**caractérisée en ce que**
l'arc dépasse en direction postérieure au-delà de la position naturelle de la cheville.

3. Prothèse de pied selon la revendication 1 ou 2,
**caractérisée en ce que**
l'élément de guidage (30) est agencé sur le côté inférieur du système amortisseur élastique (40) et est monté fermement sur le composant de structure (10) sous forme de ressort à lame dans la partie d'avant-pied (20) ou est monté sur la structure de soutien (10) de façon librement mobile autour d'un axe (50) orienté transversalement à l'extension longitudinale de la prothèse de pied.

4. Prothèse de pied selon la revendication 3,
**caractérisée en ce que**
l'élément de guidage (30) est réalisé sous forme de ressort à lame.

5. Prothèse de pied selon l'une des revendications précédentes,
**caractérisée en ce que**
le système amortisseur élastique (40) est réalisé sous forme d'élément en mousse ou d'élément en élastomère.

6. Prothèse de pied selon la revendication 5,
**caractérisée en ce que**
le système amortisseur élastique (40) est agencé de façon réversible entre le côté inférieur de la structure de soutien (10) et le côté supérieur de l'élément de guidage (30).

7. Prothèse de pied selon l'une des revendications précédentes,
**caractérisée en ce que**
un élément de plante (60) en un matériau élastique est agencé sur le côté inférieur de l'élément de guidage (30).

8. Prothèse de pied selon l'une des revendications précédentes,
**caractérisée en ce que**
la partie d'avant-pied (20) est réalisée sous forme de ressort à lame ou comprend au moins un ressort à lame.

9. Prothèse de pied selon l'une des revendications précédentes,
**caractérisée en ce que**
une butée anti-surcharge (70) est agencée entre l'élément de guidage (30) et la structure de soutien (10).

10. Prothèse de pied selon l'une des revendications précédentes,
**caractérisée en ce que**
l'élément de guidage (30) est monté de façon immobile autour d'un axe s'étendant en direction antérieure-postérieure et/ou autour d'un axe s'étendant en direction proximale-distale.

11. Prothèse de pied selon l'une des revendications précédentes,
**caractérisée en ce que**
l'extrémité postérieure de l'élément de guidage représente une extrémité libre, et un décrochement (35) est agencé en direction proximale ou réalisé sous forme de bombement convexe à l'extrémité libre de l'élément de guidage (30).

12. Prothèse de pied selon l'une des revendications précédentes,
**caractérisée en ce que**
le composant de structure (10) est réalisé de façon élastique et d'un seul tenant.

13. Prothèse de pied selon l'une des revendications précédentes,
**caractérisée en ce que**
un élément de coopération de forme (5, 6) respectif est immobilisé à l'extrémité postérieure de l'élément de guidage (30) et sur le côté inférieur du composant de structure (10), pour immobiliser le système amortisseur élastique (40).

14. Prothèse de pied selon l'une des revendications précédentes,
**caractérisée en ce que**
l'arc dépasse en direction antérieure-postérieure au-delà du système amortisseur élastique d'une longueur qui est comprise entre 10 % et 30 %, de préférence entre 12,5 % et 25 % de la longueur du pied F_{L}.

15. Prothèse de pied selon l'une des revendications précédentes,
**caractérisée en ce que**
un rembourrage (90) formant un contour est agencé sur le côté supérieur du composant de structure (10) dans la zone d'avant-pied (20).

16. Prothèse de pied selon l'une des revendications précédentes,
**caractérisée en ce que**
à partir des moyens de raccordement proximaux (2), le composant de structure (10) est incurvé de façon concave dans l'arc et présente dans la zone du soutien du système amortisseur élastique une courbure convexe avec un rayon de courbure plus grand par comparaison à l'arc, et à la suite de nouveau une courbure concave dans la partie d'avant-pied (20).

17. Prothèse de pied selon l'une des revendications précédentes,
**caractérisée en ce que**
l'élément de guidage (30) est réalisé sous la forme d'un ressort à lame ayant une forme concave - convexe - concave depuis le talon jusqu'à la pointe du pied.

18. Prothèse de pied selon l'une des revendications précédentes,
**caractérisée en ce que**
la partie d'avant-pied (20) et l'élément de guidage (30) sont associés l'un à l'autre par l'intermédiaire d'un moyen d'orientation (4).

19. Prothèse de pied selon la revendication 18,
**caractérisée en ce que**
la partie d'avant-pied (20) et l'élément de guidage (30) et le moyen d'orientation (4) sont collés les uns aux autres.

20. Prothèse de pied selon l'une des revendications précédentes,
**caractérisée en ce que**
celle-ci est réalisée pour être disposée dans une chaussure.

21. Prothèse de pied selon l'une des revendications précédentes,
**caractérisée en ce que**
l'élément de guidage (30) comprend une charnière en film.
